(19) Europäisches Patentamt
European Patent Office
Office européen des brevets

(11) EP 3 932 967 A1

(12) # EUROPÄISCHE PATENTANMELDUNG

(43) Veröffentlichungstag:
**05.01.2022 Patentblatt 2022/01**

(21) Anmeldenummer: **20183957.8**

(22) Anmeldetag: **03.07.2020**

(51) Int Cl.:
***C08G 18/73*** *(2006.01)* ***C08G 18/48*** *(2006.01)*
***C08G 18/10*** *(2006.01)* ***C08G 18/32*** *(2006.01)*
***C09D 175/02*** *(2006.01)* ***C09D 175/08*** *(2006.01)*
***C08G 18/22*** *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Technische Universität Wien**
**1040 Wien (AT)**

(72) Erfinder:
- **Ehrmann, Katharina**
  **1090 Wien (AT)**
- **Baudis, Stefan**
  **1230 Wien (AT)**
- **Liska, Robert**
  **2123 Schleinbach (AT)**

(74) Vertreter: **Ellmeyer, Wolfgang**
**Patentanwalt**
**Mariahilferstrasse 50**
**1070 Wien (AT)**

(54) **THERMOPLASTISCHE POLY(URETHAN-HARNSTOFF)-POLYADDUKTE**

(57) Die Erfindung betrifft ein thermoplastisches Poly(urethan-harnstoff)-Polyaddukt mit sterisch gehinderten Harnstoffgruppen der nachstehenden Formel (I):

$$-[I\text{-}M\text{-}(I\text{-}C_1)a\text{-}(I\text{-}M)_b\text{-}(I\text{-}C_2)c]n- \qquad (I)$$

worin I, M, $C_1$ und $C_2$ jeweils für zweiwertige Reste stehen, die jeweils über eine Urethan- oder eine Harnstoffgruppierung miteinander verbunden sind, wovon
I jeweils unabhängig für einen zweiwertigen, von einem Diisocyanat abgeleiteten Rest mit 1 bis 20 Kohlenstoffatomen steht;
M jeweils unabhängig für einen zweiwertigen, von einem Makrodiol abgeleiteten Rest eines aliphatischen Polyethers, Polyesters oder Polycarbonats mit einem zahlenmittleren Molekulargewicht $M_n \geq 500$ steht;
$C_1$ jeweils unabhängig für einen zweiwertigen, von einem Diamin oder Aminoalkohol mit jeweils zumindest einer sterisch gehinderten sekundären Aminogruppe abgeleiteten Rest mit 1 bis 30 Kohlenstoffatomen steht;
$C_2$ jeweils unabhängig für einen zweiwertigen, von einem von einem Diol, Diamin oder Aminoalkohol abgeleiteten Rest mit 1 bis 20 Kohlenstoffatomen steht;
wobei zumindest einer der Reste I, M, $C_1$ und $C_2$ eine oder mehrere Estergruppierungen umfasst; und
a, b und c jeweils unabhängig für eine ganze Zahl von 0 bis 10 stehen und n eine Zahl ≥ 3 ist, die für die Anzahl der Blöcke des Polyaddukts steht;
mit der Maßgabe, dass jeweils innerhalb desselben Blocks a + c ≥ 1 ist und in allen Blöcken zusammen zumindest ein a ≥ 1 ist und zumindest ein c ≥ 1 ist.

EP 3 932 967 A1

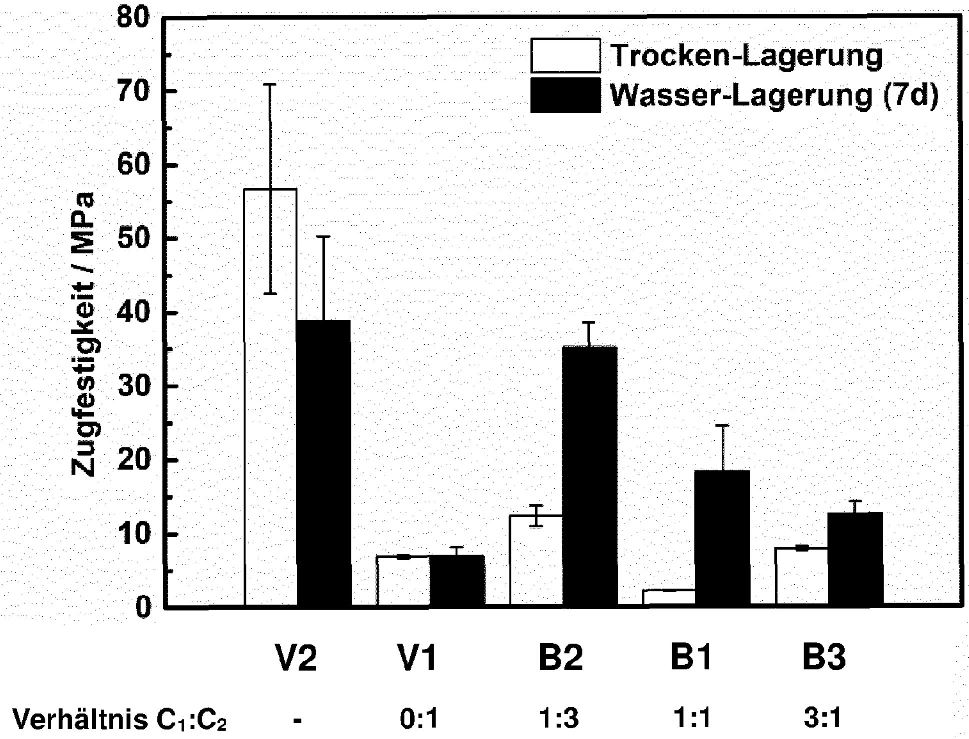
80
70
60
50
40
30
20
10
0
Zugfestigkeit / MPa
Trocken-Lagerung
Wasser-Lagerung (7d)
V2
V1
B2
B1
B3
Verhältnis C1:C2
-
0:1
1:3
1:1
3:1

Figur 2

**Beschreibung**

Die vorliegende Erfindung betrifft neue thermoplastische Poly(urethan-harnstoff)-Polyaddukte.

STAND DER TECHNIK

Für zahlreiche Verarbeitungsprozesse von Polymeren, wie z.B. Extrusion, Spritzguss, Blasformen, Lösungsgießen ("solution casting"), Rotationsbeschichtung ("spin coating") und andere thermische und lösungsbasierte Fertigungsverfahren, sind niedrige Viskositäten der Polymerschmelzen oder -lösungen bei möglichst niedrigen Temperaturen bzw. gute Löslichkeit der Polymere in gängigen Lösungsmitteln grundlegende Voraussetzungen. Polymere, die über diese wünschenswerten Eigenschaften verfügen, sind jedoch oftmals für ihre spätere Verwendung nicht ausreichend mechanisch belastbar, da zur Erreichung dieser Eigenschaften vor der Verarbeitung praktisch keine chemische Vernetzung und nur in begrenztem Ausmaß eine physikalische Vernetzung vorliegen darf. Beispielsweise sind Thermoplaste aufgrund ihrer in der Regel niedrigen Schmelzviskosität bei niedrigen bis moderaten Temperaturen für die obigen Verarbeitungsverfahren zwar prinzipiell gut geeignet, sind allerdings ebenfalls nur bedingt mechanisch belastbar.

Zur Verbesserung der physikalischen Eigenschaften dieser Polymere wird daher häufig eine Nachbearbeitung zur nachträglichen Einführung von chemisch oder physikalisch induzierter Vernetzung in die bereits verarbeiteten Polymere durchgeführt. Allerdings erfordern letztere Vernetzungsverfahren (z.B. thermisch oder Licht-basiert) große Mengen an Energie und/oder teure Ausrüstung, und für chemische Verfahren (z. B. Gas- oder Flüssigphasen-Vernetzung) werden üblicherweise Chemikalien (z. B. Thiole oder Alkalimetallhydride) eingesetzt, die mitunter kostspielig sind und bei deren Verwendung verschiedenste Sicherheitsauflagen erfüllt werden müssen.

Zu Polymeren, die oftmals über recht gute mechanische Eigenschaften verfügen, zählen beispielsweise Polyurethan-Polyharnstoff-Thermoplaste, die üblicherweise mittels Polyaddition von Diolen und Diaminen mit Diisocyanaten hergestellt werden und hierin in der Folge als thermoplastische Poly(urethan-harnstoff)-Addukte oder kurz TPUUs ("thermoplastic polyurethane-ureas") bezeichnet werden. Die Struktur solcher Addukte kann allgemein durch die nachstehende Formel (1) dargestellt werden:

$$-[(I\text{-}A)_a\text{-}(I\text{-}B)_b]_n- \qquad (1)$$

worin I, A und B jeweils für zweiwertige Reste stehen, die von einem Diisocyanat (I), einem Diol (A) bzw. einem Diamin (B) abgeleitet und jeweils über eine Urethan- oder eine Harnstoffgruppierung miteinander verbunden sind, a und b für die Anzahl der Wiederholungen der Urethan- bzw. Harnstoff-Einheiten stehen und n für die Anzahl an die beiden umfassenden Blöcken steht. Eine beispielhafte detailliertere Darstellung einer Struktur mit jeweils alternierenden Diol-Einheiten A und Diamin-Einheiten B, d. h. mit a = b = 1 ist die nachstehende Formel (2):

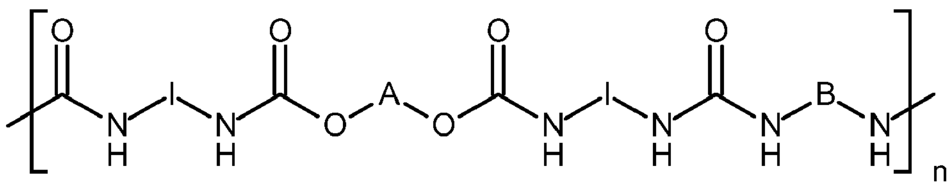

(2)

Darin wechseln sich zudem je zwei von der Addition eines Diols HO-A-OH an zwei Diisocyanat-Moleküle stammende Urethan-Gruppierungen -NH-CO-O- bzw. -O-CO-NH- mit je zwei von der Addition eines Diamins $H_2N$-B-$NH_2$ an zwei Diisocyanate stammenden Harnstoff-Gruppierungen -NH-CO-NH- ab. Stehen a und b jeweils für Zahlen > 1, so wechseln sich innerhalb eines Blocks Polyurethan- mit Polyharnstoff-Segmenten ab.

Etwas komplexer ist die Darstellung bei Verwendung von sekundären Diaminen zur Ausbildung der Harnstoff-Einheiten, da in diesen Fällen neben dem Rest B auch die beiden weiteren, an die Stickstoffatome gebundenen Reste zu erfassen sind. Beispielsweise würde ein N,N'-Dimethyl-Derivat des obigen Diamins eine Struktur gemäß nachstehender Formel (3) ergeben:

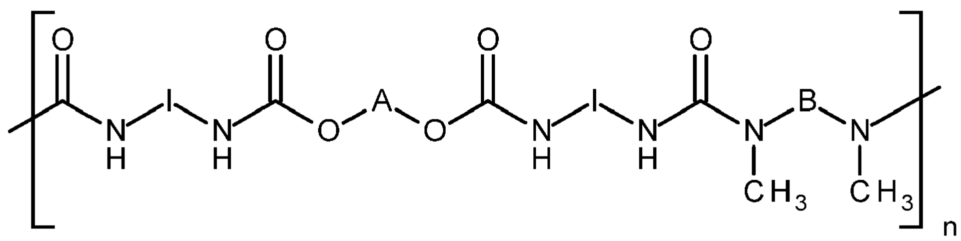

(3)

In vereinfachter Schreibweise könnte die allgemeine Struktur angegeben werden, indem der durch Entfernung der beiden N-gebundenen Wasserstoffatome des Diamins erhaltene zweiwertige Rest $-NCH_3-B-NCH_3-$ als Rest D bezeichnet wird, wie in Formel (4) dargestellt:

$$-[(I-A)_a-(I-D)_b]_n- \qquad (4)$$

Eine Kombination beider Darstellungsarten ergibt in diesem Fall eine Struktur der folgenden Formel (5):

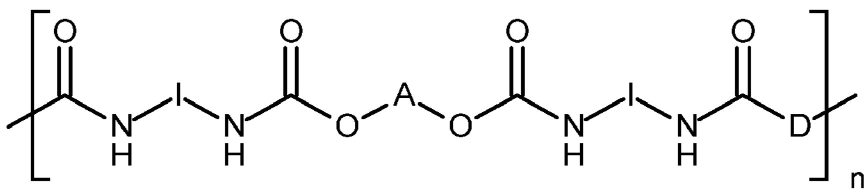

(5)

worin die beiden Stickstoffatome des Rests D jeweils eine N-methylierte HarnstoffGruppierung vervollständigen.

Die mechanischen Eigenschaften von TPUUs lassen sich durch geeignete Auswahl der Monomere in relativ weiten Bereichen steuern, z. B. durch Vorsehen eines geeigneten Verhältnisses zwischen Hart- und Weichsegmenten innerhalb der Polymerketten. So wird beispielsweise in der EP 452.775 A2 die Herstellung von TPUUs mit erhöhter Wärmebeständigkeit unter Verwendung von 4,4'-Diisocyanatodicyclohexylmethan und gegebenenfalls methyliertem Piperazin zur Herstellung von Polyharnstoff-Hartsegmenten offenbart, die mit aus Makrodiolen hergestellten Polyurethan-Weichsegmenten kombiniert werden.

Weiters ist bereits seit Jahrzehnten bekannt, dass in sterisch gehinderten Harnstoffmolekülen die Stabilität der Bindung zwischen mit zumindest einem voluminösen Rest substituierten Stickstoffatomen und der Carbonylgruppe instabil ist. So wurde bereits 1974 die Dissoziation von Harnstoffmolekülen mit verschiedenen sperrigen Substituenten beschrieben, darunter diverse Kombinationen aus Isopropyl-, sec-Butyl, tert-Butyl-, 3-Pentyl-, Cyclohexyl- und Isooctyl-Substituenten an demselben Stickstoffatom sowie eine zyklische Variante unter Verwendung von Tetramethylpiperidin (Stowell, J. C., Padegimas, S. J., "Urea dissociation. Measure of steric hindrance in secondary amines", J. Org. Chem. 39(16), 2448-2449 (1974)).

Die dabei auftretende Gleichgewichtsreaktion kann am Beispiel einer doppelten Substitution mit tert-Butyl wie im folgenden Schema A dargestellt werden:

Schema A

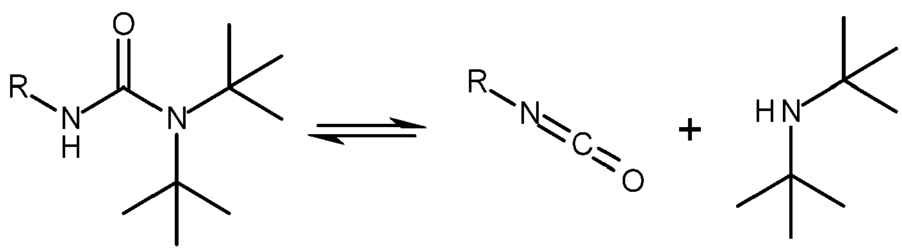

Dieses Schema veranschaulicht, dass die instabile Bindung zwischen der Carbonylgruppe und dem sterisch gehinderten Stickstoffatom des sekundären Amins unter bestimmten Bedingungen unter Ausbildung eines Isocyanats und des freien sekundären Amins reversibel gespalten wird, d. h. es bildet sich auch wieder der ursprüngliche Harnstoff zurück, wobei die Lage des Gleichgewichts maßgeblich von der Auswahl der beiden Substituenten am sterisch gehinderten Stickstoffatom abhängt.

Sterisch gehinderte Harnstoffgruppen wurden in der Folge unter anderem zur Maskierung von Isocyanaten verwendet, wobei beispielsweise Hutchby et al. vor einigen Jahren die Methanolyse von mit Methyl, Ethyl, Isopropyl und tert-Butyl N-substituierten Harnstoffen bei Temperaturen zwischen 20 °C und 70°C untersucht haben (Hutchby, M., Houlden, C. E., Ford, J. G., Tyler, S. N. G., Gagne, M. R., Lloyd-Jones, G. C., Booker-Milburn, K.I., "Hindered ureas as masked isocyanates: facile carbamoylation of nucleophiles under neutral conditions", Angew. Chem. Int. Ed. 48(46), 8721-8724 (2009)).

Die erstmalige Nutzung dieser Reaktion in Makromolekülen für die Entwicklung "dynamischer" Materialien wurde von Ying et al. beschrieben (Ying, H., Zhang, Y., Cheng, J., "Dynamic urea bond for the design of reversible and self-healing polymers", Nat. Commun. 5,3218 (2014)). Dabei wurden für molekulare Kinetikstudien 2,2,6,6-Tetramethylpiperidinylcarbonsäureamid und 1-tert-Butyl-1-isopropyl-, 1-tert-Butyl-1-ethyl-, 1,1-Diisopropyl- und 1,1-Diethylharnstoff eingesetzt. Weiters wurden ein einfaches Polyharnstoff-Molekül aus 1,3-Bis(isocyanatomethyl)cyclohexan und N,N'-Di-tert-butylethylen-diamin sowie vernetzte Polyurethan-harnstoff-Polymere aus Triethanolamin, Hexamethylendiisocyanat, Tetraethylenglykol und vier verschiedenen, sterisch gehinderten Diaminen hergestellt und untersucht, wobei sog. "selbstheilende Effekte" des vernetzten Polymermaterials festgestellt werden konnten, die jedoch stets mit einer Abnahme der Zugfestigkeit durch die Selbstheilung einhergingen.

In der Folge wurden zahlreiche weitere Experimente mit entsprechenden "selbstheilenden" Polymeren durchgeführt. So verwendeten etwa Zhang et al. verschiedene Ethanolamine, nämlich 2-tert-Butylaminoethanol, 2-Isopropylaminoethanol und N-Butylaminoethanol, zur Herstellung von sog. "recycelbaren" Poly(urethan-harnstoff)-Duroplasten (Zhang, Y., Ying, H., Hart, K. R., Wu, Y., Hsu, A. J., Coppola, A. M., Kim, T. A., Yang, K., Sottos, N. R., White, S. R., Cheng, J., "Malleable and recyclable poly-(urea-urethane) thermosets bearing hindered urea bonds", Adv. Mater. 28(35), 7646-7651 (2016)). Die recycelten Polymermaterialien erreichten dabei jedoch bestenfalls die ursprünglichen Zugfestigkeitswerte. Weiters beschrieben Zhang et al. erneut die Verwendung von Ethanolaminen als Kettenverlängerer für dynamische Poly(alkylharn-stoff-urethan)-Netzwerke mit verbessertem Spannungsabbau (Zhang, L., Rowan, S. J., "Effect of sterics and degree of cross-linking on the mechanical properties of dynamic poly(alkylurea-urethane) networks", Macromolecules 50(13), 5051-5060 (2017)). Bruce und Lewis untersuchten Glasübergangstemperaturen von selbstheilenden Poly-(urethan-harnstoffen) zur Herstellung von weniger weichen Materialien, die denselben Selbstheilungsmechanismus aufweisen (Bruce, A. C., Lewis, C. L., "Influence of glass transition temperature on mechanical and self-healing behavior of polymers bearing hindered urea bonds", SPE ANTEC, Anaheim, 2017; Anaheim, 2017), und zwei weitere Artikel betreffen die Verwendung von sterisch gehinderten Diaminen (v. a. N,N'-Di-tert-butylethylendiamin) als Kettenverlängerer zur Herstellung von reprozessierbaren Poly(urethan-harnstoff)-Duroplasten mit Formgedächtnis (Fang, Z., Zheng, N., Zhao, Q., Xie, T., "Healable, reconfigurable, reprocessable thermoset shape memory polymer with highly tunable topological rearrangement kinetics", ACS Appl. Mater. Interfaces 9(27), 22077-22082 (2017); Wang, Y., Pan, Y., Zheng, Z., Ding, X., "Reconfigurable and reprocessable thermoset shape memory polymer with synergetic triple dynamic covalent bonds", Macromol. Rapid Commun. 39(10), 1800128 (2018)).

Auch in der Patentliteratur finden sich natürlich entsprechende Offenbarungen zu solchen "selbstheilenden" Polymeren, vor allem auch von den obigen Autoren als Erfinder; siehe z. B. WO 2014/144539 A2, WO 2016/069582 A1, WO 2016/126103 A1 und WO 2016/126756 A1.

Das bei der in Schema A dargestellten Reaktion entstehende Isocyanat erfährt jedoch in wässrigem Milieu eine Hydrolyse zur entsprechenden Carbaminsäure, die anschließend zu spontaner Decarboxylierung neigt, wie dies nachstehend in Schema B dargestellt ist:

Schema B

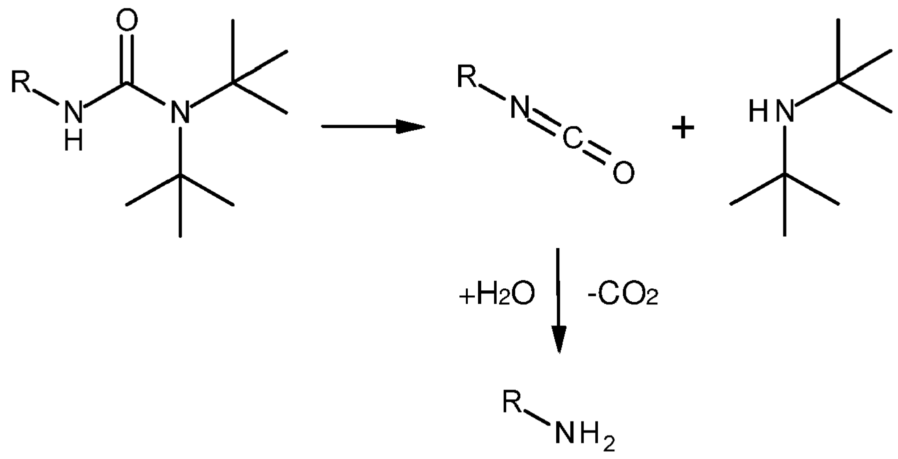


Dabei entstehen die beiden freien Amine, die ursprünglich das Harnstoff-Molekül gebildet hatten. Eine solche In-situ-Entstehung von Isocyanaten aus sterisch gehinderten Harnstoffmolekülen und anschließende Hydrolyse der Isocyanate in wässrigem Medium ermöglicht unter anderem die Hydrolyse von Polyharnstoffen.

Für Makromoleküle wurde diese Reaktionsabfolge erstmalig von Ying et al. offenbart (Ying, H., Cheng, J., "Hydrolyzable polyureas bearing hindered urea bonds", J. Am. Chem. Soc. 136(49), 16974-16977 (2014)), wobei Polyharnstoffe sowie vernetzte Poly-(urethan-harnstoff)-Organogele in DMF gelöst und anschließend mit Wasser hydrolysiert wurden, und in weiterführenden Arbeiten wurde die Biokompatibilität von Hydrogelen, die sterisch gehinderte Urethangruppen zur Hydrolyse enthielten, belegt (siehe Ying, H., Yen, J., Wang, R., Lai, Y., Hsu, J.-L.-A., Hu, Y., Cheng, J., "Degradable and biocompatible hydrogels bearing a hindered urea bond", Biomater. Sci. 5(12), 2398-2402 (2017)). Später untersuchten Cai et al. die Hydrolyse eines in THF mit 5 Vol.-% Wasser gelösten Polyharnstoffs und wiesen dabei die pH-Unabhängigkeit der Hydrolysereaktion nach (Cai, K., Ying, H., Cheng, J., "Dynamic Ureas with fast and pHindependent hydrolytic kinetics", Chem. Eur. J. 24(29), 7345-7348 (2018); sowie WO 2017/155958 A1), und kürzlich offenbarten Chen et al. die Verwendung von Polyharnstoffen mit sterisch gehinderten Harnstoffgruppen zur Wirkstofffreisetzung (Chen, M., Feng, X., Xu, W., Wang, Y., Yang, Y., Jiang, Z., Ding, J., "PEGylated polyurea bearing hindered urea bond for drug delivery", Molecules 24(8), 1538 (2019)), wobei mehrere PEGylierte Polyharnstoffe in Lösung in DMSO zur Ausbildung von Micellen rund um einen Anti-Tumor-Wirkstoff (Paclitaxel) eingesetzt wurden, eine Lösung dieser Micellen in PBS angefertigt und in Tumoren von Versuchstieren injiziert wurde, wo durch Hydrolyse der Polyharnstoff-Moleküle der Wirkstoff freigesetzt wurde.

Bei all den publizierten Versuchen zur "Selbstheilung" bzw. "Nachbehandlung" von Polyharnstoffen und Poly(urethan-harnstoffen), von denen die überwiegende Mehrzahl nur die reversible Reaktion gemäß Schema A und nur wenige auch die anschließende Hydrolysereaktion zum Gegenstand haben, konnten zwar in seltenen Fällen Teilverbesserungen einzelner mechanischer Eigenschaften erzielt werden, wie z.B. die oben erwähnten Poly(urethan-harnstoffe) (PUUs) mit verbessertem Spannungsabbau gemäß Zhang et al., zumeist wurden jedoch modifizierte Polymere erhalten, die schlechtere Eigenschaften als davor oder bestenfalls gleich gute aufwiesen. Zudem wurden noch niemals speziell thermoplastische Polyharnstoffe, Polyurethane oder Poly(urethan-harnstoff)-Polyaddukte (TPUUs) unter diesem Aspekt untersucht, und folglich wurde auch in keinem einzigen Fall über modifizierte Polymere mit Eigenschaften berichtet, die sich für die eingangs erwähnten thermomechanischen Verarbeitungsprozesse besser eignen würden als das jeweilige Ausgangsmaterial.

Dazu kommt, dass für bestimmte Anwendungsgebiete von Thermoplasten eine Kombination verschiedenster wünschenswerter Eigenschaften vorteilhaft wäre, wie z.B. gute thermomechanische Eigenschaften des Polymermaterials, sowohl vor als auch nach dessen Verarbeitung, gute Löslichkeit des Ausgangsmaterials, um lösungsbasierte Verarbeitungsverfahren zu ermöglichen oder zu vereinfachen, sowie mitunter auch Biokompatibilität des Materials, um dessen Einsetzbarkeit in Kontakt mit dem menschlichen oder tierischen Körper zu gewährleisten. TPUUs, die sich durch derartige Eigenschaftskombinationen auszeichnen, sind allerdings bislang gänzlich unbekannt.

Ziel der Erfindung war daher die Entwicklung eines neuen thermoplastischen Poly-(urethan-harnstoff)- (TPUU-) Materials mit selbstheilenden Eigenschaften auf Basis von sterisch gehinderten sekundären Aminen, das nach der Reaktion in wässrigem Milieu verbesserte thermomechanische Eigenschaften aufweist und daher zur Verwendung in entsprechenden Verarbeitungsverfahren besser geeignet ist als bekannte Materialien nach dem Stand der Technik und das darüber hinaus gute Löslichkeit sowie Biokompatibilität aufweist, um für einen Einsatz in biomedizinischen Anwendungen geeignet zu sein.

OFFENBARUNG DER ERFINDUNG

Dieses Ziel erreicht die vorliegende Erfindung durch Bereitstellung eines thermoplastischen Poly(urethan-harnstoff)- (TPUU-) Polyaddukts mit sterisch gehinderten Harnstoffgruppen der nachstehenden Formel (I):

$$-[I-M-(I-C_1)_a-(I-M)_b-(I-C_2)_c]_n- \qquad (I)$$

worin I, M, $C_1$ und $C_2$ jeweils für zweiwertige Reste stehen, die jeweils über eine Urethan- oder eine Harnstoffgruppierung miteinander verbunden sind, wovon

I jeweils unabhängig für einen zweiwertigen, von einem Diisocyanat abgeleiteten, gesättigten oder ungesättigten, aliphatischen, alicyclischen oder aromatischen Rest mit 1 bis 20 Kohlenstoffatomen steht;

M jeweils unabhängig für einen zweiwertigen, von einem Makrodiol abgeleiteten Rest eines aliphatischen Polyethers, Polyesters oder Polycarbonats mit einem zahlenmittleren Molekulargewicht $M_n \geq 500$ steht;

$C_1$ jeweils unabhängig für einen zweiwertigen, von einem Diamin oder Aminoalkohol mit jeweils zumindest einer sterisch gehinderten sekundären Aminogruppe durch Entfernung je eines N-gebundenen Wasserstoffatoms des Diamins oder eines N-gebundenen und des O-gebundenen Wasserstoffatoms des Aminoalkohols abgeleiteten, gesättigten oder ungesättigten, aliphatischen oder alicyclischen Rest mit 1 bis 30 Kohlenstoffatomen steht;

$C_2$ jeweils unabhängig für einen zweiwertigen, von einem von einem Diol, Diamin oder Aminoalkohol abgeleiteten, gesättigten oder ungesättigten, aliphatischen, alicyclischen oder aromatischen Rest mit 1 bis 20 Kohlenstoffatomen steht;

wobei in den Resten von I, $C_1$ und $C_2$ bei Vorliegen von mehr als vier Kohlenstoffatomen gegebenenfalls zumindest eines davon durch ein aus Sauerstoff und Stickstoff ausgewähltes Heteroatom ersetzt ist;

wobei zumindest einer der Reste I, M, $C_1$ und $C_2$ eine oder mehrere Estergruppierungen umfasst; und

a, b und c jeweils unabhängig für eine ganze Zahl von 0 bis 10 stehen und n eine Zahl $\geq 3$ ist, die für die Anzahl der Blöcke des Polyaddukts steht;

mit der Maßgabe, dass jeweils innerhalb desselben Blocks $a + c \geq 1$ ist und in allen Blöcken zusammen zumindest ein $a \geq 1$ ist und zumindest ein $c \geq 1$ ist.

Die Erfinder des vorliegenden Anmeldungsgegenstands haben für solche thermoplastische Poly(urethan-harnstoff)- (TPUU-) Polyaddukte mit sterisch gehinderten Harnstoffgruppen überraschenderweise herausgefunden, dass diese Makromoleküle nicht nur in Lösung oder als Hydrogel, wie dies aus dem Stand der Technik bekannt war, sondern auch im festen Zustand bei Kontakt mit Wasser oder in wässriger Umgebung die in Schema B dargestellte Reaktion zeigen - und das sogar nach Verarbeitung des Materials zu festen Erzeugnissen, wie z.B. durch Lösungsgießen, Folienziehen oder dergleichen.

Darüber hinaus war jedoch noch viel überraschender, dass das durch die Öffnung der labilen Harnstoffbindung gebildete Isocyanat bei Verwendung der erfindungsgemäßen TPUUs in festem Zustand offenbar nicht, wie zuvor in Schema B gezeigt, vollständig zum freien Amin hydrolysiert wird, sondern ein Teil des Isocyanats mit einem Teil des durch die Hydrolyse entstandenen freien Amins unter Ausbildung einer neuen, nicht sterisch gehinderten Harnstoffgruppierung reagiert, woraus stabile Polymerketten mit verbesserten thermomechanischen Eigenschaften resultieren, wie die späteren Beispiele belegen. Ohne sich auf eine bestimmte Theorie festlegen zu wollen, nehmen die Erfinder an, dass das Auftreten dieser - hierin als "Rekombinationsreaktion" bezeichneten - Reaktion eines decarboxylierten Isocyanats mit einem noch nicht decarboxylierten auf die verringerte Reaktionsgeschwindigkeit der Hydrolysereaktion der Polymerketten in der festen Phase zurückzuführen ist. Im nachstehenden Schema C ist eine solche Reaktionsabfolge - analog zu obigem Schema B - für eine endständige, sterisch gehinderte Harnstoffgruppe dargestellt:

Schema C

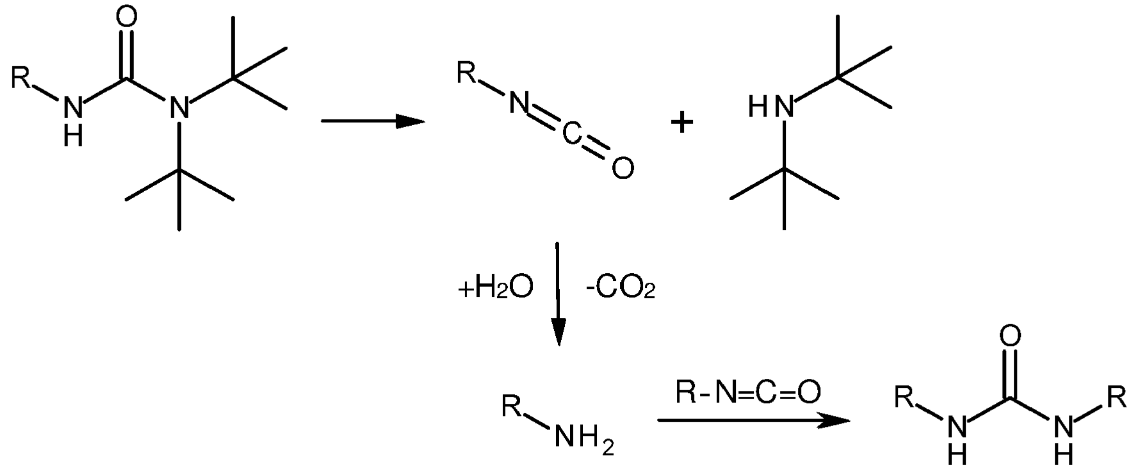


Und im folgenden Schema D für zwei Harnstoffgruppierungen inmitten einer TPUU-Polymerkette, die von demselben, beidseitig sterisch gehinderten sekundären Diamin, im vorliegenden Fall von N,N'-Di-tert-butylpropylendiamin, herrühren:

Schema D

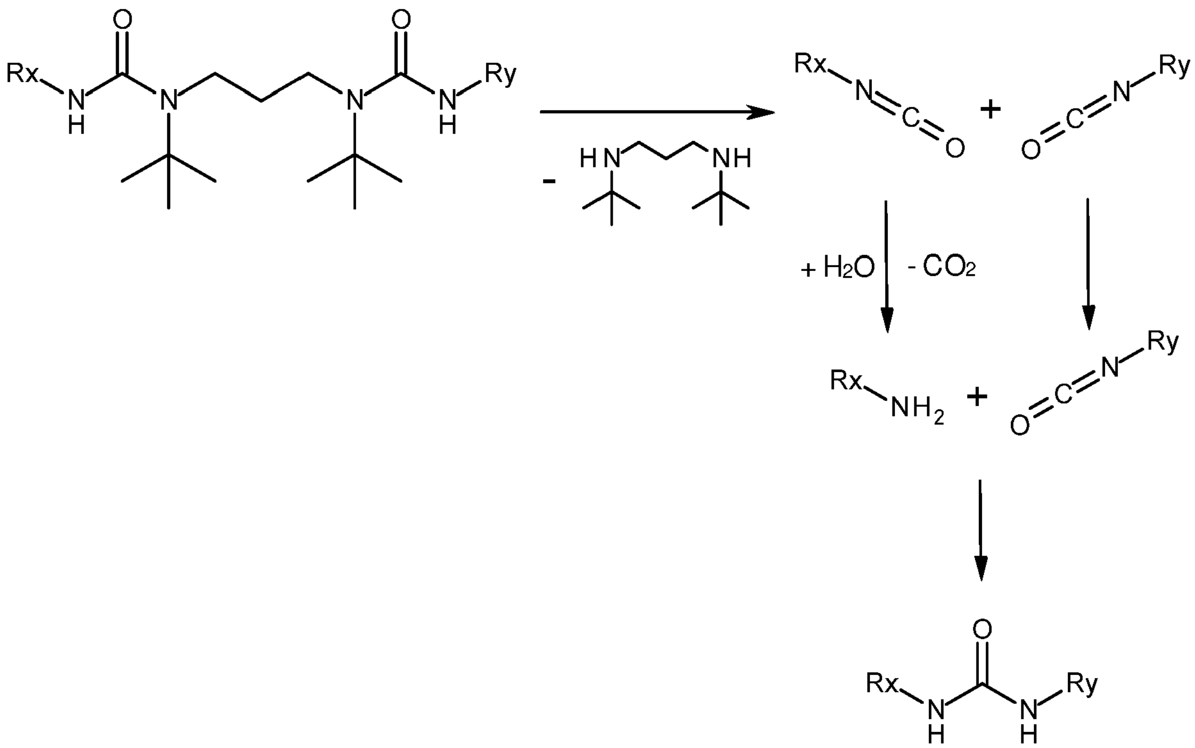


Auf diese Weise bildet sich bei Kontakt des TPUUs mit Wasser ein neues TPUU-Polymer, das formal aus der Eliminierung eines beidseitig sterisch gehinderten Diamins und einer Carbonylgruppe aus der Hauptkette des Polymers resultiert.

Die Reaktion von TPUUs, die Reste von nur einseitig sterisch gehinderte Diaminen oder Aminoalkoholen enthalten, ist im umseitigen Schema E dargestellt, worin X für O (von einem Aminoalkohol stammend) oder NH (von einem Diamin stammend) steht:

Schema E

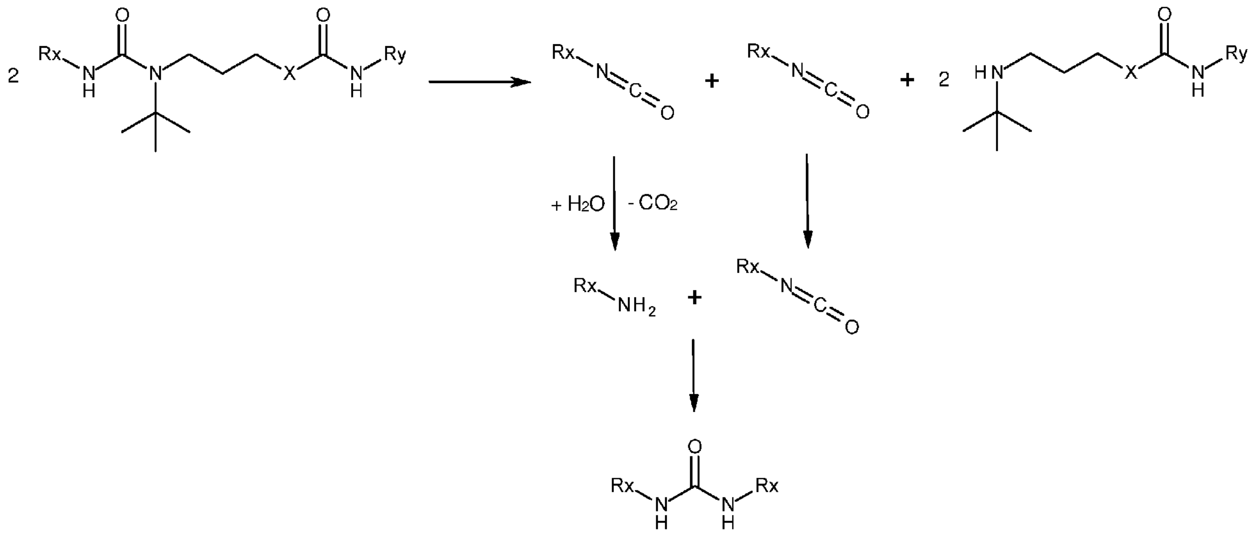


Hier entstehen durch Bindungsöffnung jeweils einer sterisch gehinderten Harnstoffgruppierung zweier TPUU-Polymerketten zwei polymere Isocyanate Rx-N=C=O, von denen eines in Gegenwart von Wasser die bekannte Hydrolyse und Decarboxylierung zum freien Amin Rx-$NH_2$ erfährt, das jedoch in der Folge an das zweite, noch nicht decarboxylierte Isocyanat addiert und dadurch unter Verdoppelung der Kettenlänge des Rests Rx-NH- ein neues, stabiles TPUU-Polymer bildet. Weiters entstehen zwei, gegenüber dem Ausgangsmolekül um die Länge von Rx verkürzte neue TPUU-Polymere mit endständigen, sterisch gehinderten sekundären Aminogruppen, die zwar gegebenenfalls mit einem der Isocyanate die aus dem Stand der Technik bekannte, "selbstheilende" Rückreaktion zum instabilen Ausgangspolymer eingehen, allerdings nicht in der Lage sind, mit Isocyanaten stabile Harnstoffbindungen auszubilden.

Auf diese Weise bilden sich aus den erfindungsgemäßen TPUUs in festem Zustand in wässrigem Milieu jeweils neue Polymere, die je nach der Lage der sich öffnenden instabilen Bindung(en) innerhalb der Hauptketten größere Kettenlängen (Schema C), im Wesentlichen dieselben (Schema D) oder sowohl größere als auch kürzere Kettenlängen (Schema E) Kettenlängen als die Ausgangsmoleküle aufweisen können, die jedoch in allen Fällen nach der Reaktion mit Wasser ausschließlich stabile, nicht sterisch gehinderte Harnstoffbindungen umfassen.

Im Gegensatz dazu entstehen aus den in der Vergangenheit untersuchten wässrigen Lösungen von Polyharnstoffen ausschließlich kürzere Moleküle, da die Hydrolyse und anschließende Decarboxylierung der durch die Öffnung instabiler, sterisch gehinderter Harnstoffbindungen gebildeten Isocyanate in Lösung viel rascher abläuft, so dass diese nicht ausreichend lange stabil sind, um eine Reaktion mit einem freien Amin einzugehen. Die im obigen Schema D dargestellte exemplarische Reaktion von TPUUs gemäß vorliegender Erfindung in festem Zustand bei Kontakt mit Wasser, die zu einem beidseitig sterisch gehinderten, freien sekundären Diamin sowie einer "rekombinierten" und nur um die Länge des Diamins und einer Carbonylgruppe verkürzten Polyharnstoffkette führt, würde in Lösung neben dem freigesetzten sterisch gehinderten Diamin lediglich zwei polymere freie Amine ergeben, zwischen denen freilich keine Reaktion erfolgen kann, wie im nachfolgenden Schema F dargestellt:

Schema F

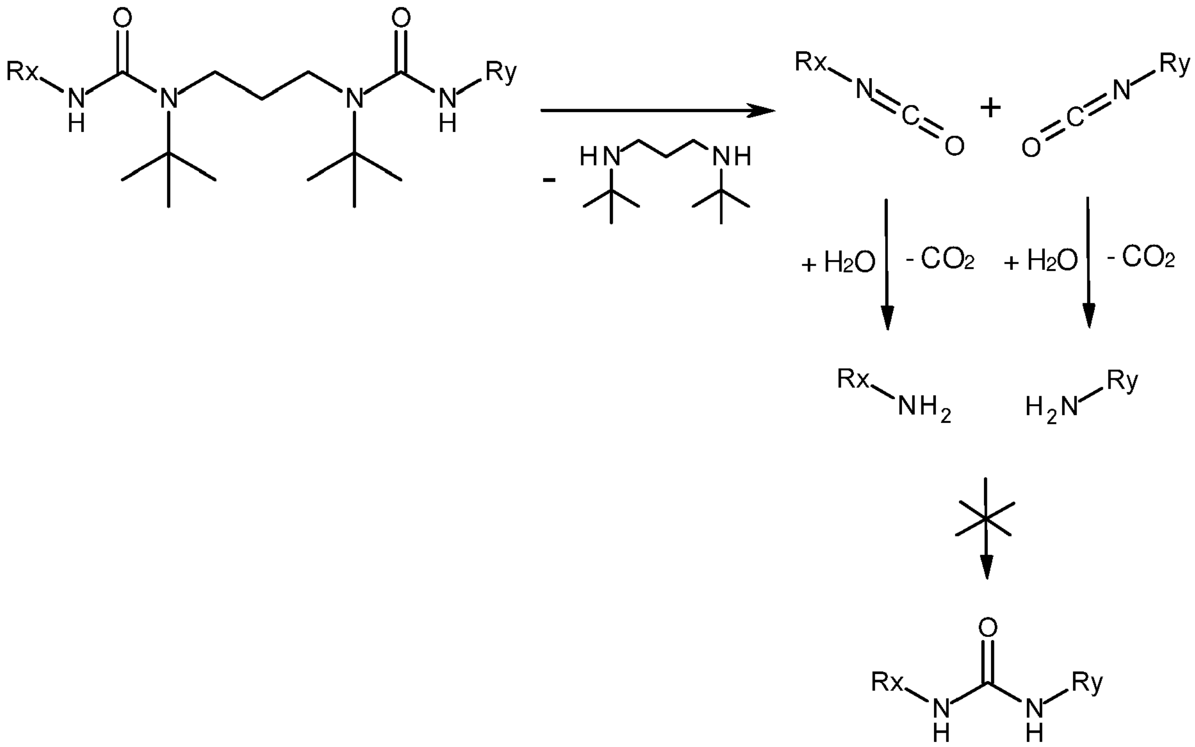


Dabei entstehen zwei neue Polymere, deren Molekulargewichte, je nach Position des vom sterisch gehinderten sekundären Diamin stammenden Rests innerhalb der ursprünglichen Polymerkette, geringer sind als jenes des Ausgangspolymers und das mitunter deutlich, bis hin zu einer etwa 50%igen Reduktion. Auch aus diesem Grund wurden in nahezu allen einschlägigen Dokumenten des Standes der Technik, die über das Verhalten von "selbstheilenden" Polyharnstoffen in wässriger Lösung berichten, bestenfalls gleichbleibende, zumeist aber verschlechterte thermomechanische Eigenschaften der entstandenen kürzeren Polymere festgestellt.

Darüber hinaus weisen die gemäß obigem Schema D aus erfindungsgemäßen TPUUs in festem Zustand bei Kontakt mit Wasser (oder einem wässrigen Milieu) erhaltenen neuen Polymere Rx-NH-CO-NH-Ry jedoch - unter der Annahme, dass in der Polymerkette nur ein sterisch gehindertes Diamin enthalten war - nicht nur praktisch dasselbe Molekulargewicht wie vor der Behandlung mit Wasser auf. Vielmehr enthalten sie vor allem auch eine neue Harnstoffgruppierung, deren beiden Wasserstoffatome nicht durch voluminöse Reste abgeschirmt sind und daher zur Ausbildung von Wasserstoffbrücken zu Harnstoff- oder Urethangruppierungen einer weiteren, benachbarten Polymerkette in der Lage sind. Ohne sich auf eine Theorie festlegen zu wollen, sind diese Wasserstoffbrücken nach Ansicht der Erfinder der Hauptgrund für die Verbesserung der thermomechanischen Eigenschaften, da dieser Effekt - wie die späteren Beispiele belegen - überraschenderweise selbst dann auftritt, wenn eine Vielzahl von sterisch gehinderten Harnstoffgruppierungen innerhalb der Ketten der erfindungsgemäßen TPUUs enthalten ist, so dass durch die "Rekombinationsreaktionen" durchwegs neue Ketten mit (mitunter sogar deutlich) niedrigerem Molekulargewicht entstehen.

So weisen etwa die erfindungsgemäßen TPUUs bei Verarbeitung zu festen Produkten bereits nach wenigen Stunden der Lagerung der Produkte in Wasser bessere Dehnbarkeits- und Zugfestigkeitswerte sowie höhere Schmelzpunkte als unmittelbar nach der Verarbeitung auf. Gleichzeitig war jedoch die Löslichkeit vor der Wasserbehandlung höher, was eine einfachere Verarbeitbarkeit der Ausgangspolymere gewährleistet.

Dazu kommt, dass aufgrund der Gegenwart von Estergruppierungen in den Ketten der erfindungsgemäßen TPUUs sowohl die Ausgangspolymere als auch deren durch "Rekombination" gebildeten Reaktionsprodukte in einem geeigneten wässrigen Milieu, wie z.B. unter physiologischen Bedingungen, durchwegs spaltbar sind, was den Vorteil der biologischen Abbaubarkeit mit sich bringt. Daher sind aus den erfindungsgemäßen TPUUs hergestellte feste Erzeugnisse auch hervorragend für medizinische Zwecke, wie z.B. als Körperimplantate oder für andere Anwendungen, die einen zeitlich zu begrenzenden Verbleib innerhalb des Körpers erfordern, geeignet.

Die in Formel (I) enthaltenen Komponenten der erfindungsgemäßen TPUUs stehen dabei als Abkürzungen, wie in der Polyurethan- und Polyharnstoff-Chemie üblich, für Isocyanat "I", Makrodiol "M", sowie zwei unterschiedliche Arten von Amino- bzw. OH-Gruppen enthaltenden "Chain Extendern", also "Kettenverlängerern", "$C_1$" und "$C_2$", die als

Hartsegmente zur Verknüpfung von Isocyanat- und Makrodiol-Bausteinen über entsprechende Urethan- und/oder Harnstoffbindungen dienen. Von diesen beiden Kettenverlängerern dient, wie oben definiert, $C_1$ als ein- oder beidseitig sterisch gehindertes Diamin oder als Aminoalkohol mit sterisch gehinderter sekundärer Aminogruppe zur Ausbildung der instabilen und in wässriger Umgebung spaltbaren Harnstoffbindungen. Und $C_2$ dient als weiteres Hartsegment einerseits zur zusätzlichen Verknüpfung von Makrodiol-Bausteinen und somit zur Steuerung der Kettenlänge zwischen den von $C_1$ gebildeten sterisch gehinderten Harnstoffgruppierungen, andererseits aber in bevorzugten Ausführungsformen zusätzlich zur Förderung der biologischen Abbaubarkeit der erfindungsgemäßen TPUUs, indem als Monomerbausteine zur Einführung von $C_2$ in die TPUU-Kette Diamine, Diole bzw. Aminoalkohole ausgewählt werden, die eine unter physiologischen Bedingungen spaltbare Estergruppierung enthalten. Dies ist insbesondere vorteilhaft, wenn als Makrodiol ein Polyether ohne spaltbare Carboxylat- oder Carbonat-Estergruppierungen gewählt wird.

In bevorzugten Ausführungsformen ist ein erfindungsgemäßes thermoplastisches TPUU durch einen oder mehrere der folgenden Parameter gekennzeichnet:

a und c sind jeweils unabhängig $\leq 5$ oder $\leq 3$; und/oder
a und c sind jeweils unabhängig $\geq 1$; und/oder
$b \geq 1$; und/oder
b = c oder b = a oder b + 1 = a + c; und/oder
$n \geq 5$ oder $n \geq 10$ oder $n \geq 50$.

Dabei bewirken relativ niedrige Werte für a und c einen vergleichsweise hohen Anteil an den von Makrodiolen stammenden Einheiten M, d. h. an Weichsegmenten, in den TPUUs der Erfindung, was für niedrige Schmelzpunkte der Polymere und hohe Flexibilität auch bei relativ niedrigen Temperaturen sowie für eine nicht übermäßig hohe Anzahl an instabilen Harnstoffbindungen im gesamten Polymer sorgt, um bei der Reaktion der verarbeiteten TPUUs in wässriger Umgebung keine allzu kurzkettigen Reaktionsprodukte zu erhalten.

Sind sowohl a als auch c jeweils $\geq 1$, sind in jedem Block sowohl zumindest eine von $C_1$ gebildete, sterisch gehinderte Harnstoffgruppe als auch ein weiterer Kettenverlängerer $C_2$, der vorzugsweise eine spaltbare Estergruppierung umfasst, enthalten.

In TPUUs der Formel (I) mit $b \geq 1$, was vorzugsweise für den Fall, dass a und c jeweils unabhängig $\geq 1$ sind, gilt, sind die beiden Kettenverlängerer-Einheiten $C_1$ und $C_2$ durch zumindest eine Makrodiol-Einheit voneinander getrennt. Dies verbessert die Steuerbarkeit des Abstands zwischen diesen beiden Einheiten und ermöglicht in besonders bevorzugten Ausführungsformen, einen relativ großen Abstand zwischen sterisch gehinderten Harnstoffgruppierungen in $C_1$ und spaltbaren Estergruppierungen in $C_2$ vorzusehen, damit es während der Verwendung der zu festen Erzeugnissen verarbeiteten erfindungsgemäßen TPUUs und den dabei auftretenden Reaktionen der freien Amine mit nicht decarboxylierten Isocyanatgruppierungen nicht auch zu einer verfrühten Spaltung von Estergruppierungen durch Angriff der freien Amine kommt.

Ausführungsformen, in denen b = c oder b = a oder b + 1 = a + c ist, bieten vor allem Vorteile bei der Herstellung der erfindungsgemäßen TPUUs der Formel (I). So kann beispielsweise in den beiden ersten Fällen ein Oligomer oder Präpolymer mit alternierenden, mittels Diisocyanaten verknüpften Kettenverlängerer-Einheiten $C_1$ oder $C_2$ und Makrodiol-Einheiten M durch Vermischen des entsprechenden sterisch gehinderten Diamins oder Aminoalkohols (für $C_1$) oder nicht sterisch gehinderten Diamins oder Aminoalkohols oder Diols (für $C_2$) mit einer äquimolaren Mengen an Makrodiol mit einem geringfügigen molaren Überschuss an Diisocyanat hergestellt werden, bevor das Reaktionsprodukt mit den jeweils gewünschten molaren Mengen des anderen Kettenverlängerers und an Diisocyanat umgesetzt wird. Und im Fall von b + 1 = a + c können einerseits zwei solcher Oligomere oder Präpolymere, die jeweils einen Kettenverlängerer mit Makrodiol-Einheiten alternierend enthalten, auf einfache Weise getrennt voneinander hergestellt und anschließend über Diisocyanat miteinander verbunden werden. Und andererseits können in besonders bevorzugten Ausführungsformen, in denen a, b und c jeweils 1 sind, äquimolare Mengen an die beiden Kettenverlängerer $C_1$ und $C_2$ bereitstellenden Monomerbausteinen mit der doppelten Menge an Makrodiol und der vierfachen Menge an Diisocyanat (oder vorzugsweise einem geringen Überschuss an Diisocyanat) umgesetzt werden, d. h. in einem Verhältnis $C_1 : C_2 : M : I$ von 1 : 1 : 2 : 4 (oder vorzugsweise > 4, z. B. 4,02 oder 4,03), was die Synthese vereinfacht. Diese kann mitunter auch sozusagen als "Eintopf-Polyaddition" durchgeführt werden, wobei allerdings die Reaktivitäten der die kettenverlängernden Reste $C_1$ und $C_2$ in die erfindungsgemäßen TPUUs einführenden Diamine, Aminoalkohole oder Diole zu berücksichtigen sind. Bei zu großen Unterschieden in deren Reaktivität, zumal zumindest ein sterisch gehindertes Amin als Reaktant zugegen ist, werden mitunter nur verhältnismäßig kurzkettige Polyaddukte erhalten, wenn alle Reaktanten gleichzeitig vermischt werden, was in der Regel nicht zu bevorzugen ist.

Aus dieser Option der Polymerisationsreaktionsführung folgt jedoch für den Fachmann auch, dass die Reihenfolge der Komponenten innerhalb eines Blocks, insbesondere jene der Komponenten $C_1$ und $C_2$, nicht auf die in Formel (I) explizit dargestellte beschränkt ist. Das bedeutet, dass bei Werten für a und c von jeweils > 1, selbst wenn a = c ist, sich die beiden, jeweils einen der Kettenverlängerer enthaltenden Bausteine ($I$-$C_1$) und ($I$-$C_2$) innerhalb eines Blocks

nicht zwingend abwechseln müssen, sondern auch statistisch verteilt sein können.

Die Anzahl der Blöcke n der erfindungsgemäßen TPUUs und damit deren zahlenmittleres Molekulargewicht ist nicht speziell eingeschränkt und kann in Abhängigkeit vom jeweiligen Verwendungszweck und den dafür wünschenswerten thermomechanischen oder sonstigen physikalischen Eigenschaften frei gewählt werden. Wie dem Fachmann wohlbekannt ist, hängt die Kettenlänge von Polyaddukten vor allem von der Stöchiometrie der Monomer- oder Präpolymerbausteine während der Polyadditionsreaktionen ab. Vorzugsweise ist die Anzahl der Blöcke n gemäß vorliegender Erfindung $\geq 5$, noch bevorzugter $\geq 10$ und insbesondere $\geq 20$, $\geq 50$ oder $\geq 100$, um die Eignung der erfindungsgemäßen TPUUs für eine Vielzahl verschiedenster thermomechanischer oder lösungsbasierter Verarbeitungsverfahren zu gewährleisten.

In besonders bevorzugten Ausführungsformen sind die erfindungsgemäßen TPUUs dadurch gekennzeichnet, dass zumindest ein Teil der Estergruppierungen unter physiologischen Bedingungen spaltbar ist und die Reste I, M, $C_1$ und $C_2$ sowie etwaige Spaltprodukte davon biokompatibel und physiologisch unbedenklich sind. Dies ermöglicht beispielsweise ihre Verwendung zur Herstellung von Implantaten, die im Körper eines Patienten im Laufe der Zeit abgebaut werden, ohne dabei Schäden zu verursachen.

Die zur Herstellung der erfindungsgemäßen TPUUs einsetzbaren Diisocyanate sind gemäß vorliegender Erfindung nicht speziell eingeschränkt, solange die daraus resultierenden Einheiten I jeweils 1 bis 30 Kohlenstoffatome aufweisen. In bevorzugten Ausführungsformen sind diese Reste I jeweils unabhängig von einem Diisocyanat aus der aus den folgenden bestehenden Gruppe abgeleitet: 1,6-Hexamethylendiisocyanat, 4,4'-Diisocyanatodicyclohexylmethan (4,4'-Methylendi(cyclohexylisocyanat), $H_{12}$MDI), Isophorondiisocyanat, 1,3-Bis(isocyanatomethyl)cyclohexan, Diphenylmethan-4,4'-diisocyanat (4,4'-Diisocyanatodiphenylmethan; Methylendi(phenylisocyanat), MDI) und L-Lysinethylesterdiisocyanat, da diese Verbindungen vorteilhafte Strukturen aufweisen, physiologisch unbedenklich sind und zudem relativ kostengünstig im Handel erhältlich sind.

Ähnliches gilt für die Makrodiole zur Einführung der Einheiten M in die erfindungsgemäßen TPUUs. Diese sind, abgesehen von einem zahlenmittleren Molekulargewicht $M_n \geq 500$ der Einheiten M, um für geeignete Molekulargewichte sowie Thermoplastizität der Polyaddukte zu sorgen, nicht speziell eingeschränkt. Vorzugsweise sind die Reste M jedoch jeweils unabhängig von einem Polyether, Polyester oder Polycarbonat aus der aus den folgenden bestehenden Gruppe abgeleitet: Polytetrahydrofuran, Polyethylenglykol, Polypropylenglykol, Polycaprolacton, Polylactid, Polyglykolid, Poly(lactid-co-glykolid) und Polyhexamethylencarbonat, noch bevorzugter von solchen mit einem zahlenmittleren Molekulargewicht $M_n \geq 800$, insbesondere $\geq 1.000$. Diese sind physiologisch unbedenklich, allgemein im Handel erhältlich oder auf einfache Weise synthetisierbar und verfügen über wohlbekannte Eigenschaften, anhand derer sich die Eigenschaften der erfindungsgemäßen TPUUs gut steuern lassen.

Auch die zur Einführung der zweiwertigen Reste mit jeweils zumindest einer sterisch gehinderten sekundären Aminogruppe eingesetzten Diamine oder Aminoalkohole sind nicht speziell eingeschränkt, solange sie zusätzlich zu den beiden Stickstoffatomen oder dem Stickstoff- und dem Sauerstoffatom ausschließlich gesättigte oder ungesättigte, aliphatische oder alicyclische Reste mit insgesamt 1 bis 30 Kohlenstoffatomen aufweisen. Damit ist hierin die Gesamtheit der Kohlenstoffatome aller Reste gemeint, die an das oder die Stickstoffatome des Aminoalkohols oder Diamins mit jeweils (zumindest) einer sterisch gehinderten sekundären Aminogruppe gebunden sind, d. h. sowohl des die beiden Aminogruppen bzw. die Amino- und die Hydroxylgruppe verbindenden zweiwertigen Rests $R_1$ als auch des Substituenten $R_2$ am Stickstoffatom der sekundären Aminogruppe(n), wie in den nachstehenden Formeln (II) (Diamin) und (III) (Aminoalkohol) dargestellt ist:

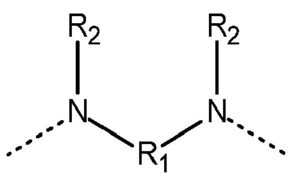

(II)

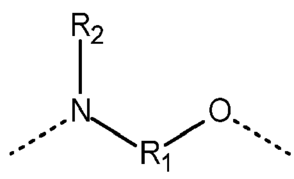

(III)

worin die gestrichelten Linien jeweils die Bindung an die Carbonylgruppe einer die Reste I, M, $C_1$ und $C_2$ verbindenden Urethan- oder Harnstoffgruppierung anzeigen und zumindest einer der Reste $R_1$ und $R_2$ ein sperriger Rest ist, der eine sterische Hinderung der Harnstoffgruppierung innerhalb des TPUUs, deren Teil das jeweilige Stickstoffatom ist, bewirkt. Im Falle von Diaminen mit nur einer sekundären Aminogruppe ist einer Reste $R_2$ in Formel (II) Wasserstoff, und im Falle von Diaminen mit zwei sterisch gehinderten sekundären Aminogruppen ist in Formel (II) entweder zumindest der Rest $R_1$ (und gegebenenfalls auch einer oder beide der Reste $R_2$) ein sperriger Rest, oder beide Reste $R_2$ (und gegebenenfalls auch der Rest $R_1$) sind sperrige Reste.

In bevorzugten Ausführungsformen der erfindungsgemäßen TPUUs sind die Reste $C_1$ allerdings nicht von Aminoalkoholen, sondern von beidseitig sterisch gehinderten sekundären Diaminen abgeleitet, um die oben in Schema D dargestellten Rekombinationsreaktionen zu ermöglichen. Noch bevorzugter sind sie aus Resten der obigen Formel (II) ausgewählt, worin zumindest die beiden Reste $R_2$ und gegebenenfalls auch der Rest $R_1$ sperrige Reste sind.

Noch bevorzugter sind sie aus Resten der obigen Formel (II) ausgewählt, worin

$R_1$ aus zweiwertigen, gesättigten oder ungesättigten, aliphatischen oder alicyclischen Resten mit 1 bis 20 Kohlenstoffatomen ausgewählt ist; und
die $R_2$ jeweils unabhängig aus einwertigen, sperrigen, gesättigten oder ungesättigten, aliphatischen oder alicyclischen Resten mit 1 bis 10 Kohlenstoffatomen ausgewählt sind;
besonders bevorzugt aus Resten der Formel (II), worin
$R_1$ aus $C_1$-$C_{10}$-Alkylen- und $C_4$-$C_{10}$-Cycloalkylenresten ausgewählt ist; und/oder
die $R_2$ jeweils unabhängig aus 1,1-Dimethyl-substituierten, gesättigten oder ungesättigten $C_1$-$C_6$-Alkylresten und 1-Methyl-substituierten $C_3$-$C_6$-Cycloalkylresten ausgewählt sind; und insbesondere aus Resten der Formel (II), worin
$R_1$ aus $C_2$-$C_6$-Alkylen- und $C_5$-$C_6$-Cycloalkylenresten ausgewählt ist; und/oder
die $R_2$ jeweils unabhängig aus Isopropyl, tert-Butyl, 1,1-Dimethylpropyl und 1-Methylcyclohexyl ausgewählt sind.

Diese bevorzugten Auswahloptionen aus Resten relativ kurzkettiger, beidseitig sterisch gehinderter sekundärer Diamine bewirkt, dass im Vergleich zu den höhermolekularen Weichsegmenten M kurzkettige Hartsegmente $C_1$ in den erfindungsgemäßen TPUUs enthalten sind, was die Thermoplastizität der Polymere fördert und in der Folge auch die Wahrscheinlichkeit der "Rekombinationsreaktion" gemäß obigem Schema D während des Kontakts von aus den TPUUs hergestellten festen Erzeugnissen mit Wasser erhöht. Darüber hinaus gelten sie ebenfalls als physiologisch unbedenklich.

Nicht speziell eingeschränkt ist weiters die Auswahl der zusätzlichen Kettenverlängerer-Einheiten $C_2$, solange sie von einem Diol, Diamin oder Aminoalkohol abgeleitete, gesättigte oder ungesättigte, aliphatische, alicyclische oder aromatische Reste mit 1 bis 20 Kohlenstoffatomen darstellen. Diese Diamine oder Aminoalkohole zur Einführung von $C_2$ in die TPUUs der Erfindung sind jedoch - im Gegensatz zu $C_1$ - keine sterisch gehinderten Amine und umfassen zudem vorzugsweise nur primäre Aminogruppen, da die Reste $C_2$ vor allem zur Einführung zusätzlicher Hartsegmente ohne instabile Harnstoffgruppierungen in die erfindungsgemäßen TPUUs dienen, um die physikalischen Eigenschaften der Polyaddukte besser steuern zu können.

In bevorzugten Ausführungsformen dienen die Reste $C_2$ allerdings auch dazu, für biologische Abbaubarkeit der Polyaddukte zu sorgen oder diese zu verbessern -je nachdem, ob einer oder mehrere der Reste I, M und $C_1$ spaltbare Estergruppierungen umfassen. Insbesondere wenn dies nicht der Fall ist, z. B. wenn M von einem Polyetherdiol abgeleitet ist, sind bevorzugte Ausführungsformen der TPUUs der vorliegenden Erfindung dadurch gekennzeichnet, dass zumindest einer der Reste $C_2$ eine oder mehrere solcher Estergruppierungen umfasst. Dabei sind die Reste $C_2$ besonders bevorzugt jeweils unabhängig von einem Diol aus der aus den folgenden bestehenden Gruppe abgeleitet: Bis(hydroxyethyl)terephthalat, Bis(hydroxypropyl)carbonat, 2-Hydroxyethyllactat und 2-Hydroxyethylglykolat, da diese relativ kurzkettig, physiologisch unbedenklich, kostengünstig im Handel erhältlich und leicht handhabbar sind.

Ein TPUU der vorliegenden Erfindung ist in besonders bevorzugten Ausführungsformen dadurch gekennzeichnet, dass b + 1 = a + c ist und das Polyaddukt der nachstehenden Formel (IV) entspricht:

$$-[(I\text{-}M\text{-}I\text{-}C_1)_a\text{-}(I\text{-}M\text{-}I\text{-}C_2)_c]_n\text{-} \qquad (IV)$$

worin

a und c jeweils unabhängig aus 1 bis 3 ausgewählt sind oder
a und c jeweils 1 sind; und
$n \geq 5$ oder $n \geq 10$ oder $n \geq 20$ ist.

Dadurch sind erfindungsgemäße TPUUs durch ein gut steuerbares Polyadditionsverfahren herstellbar. Bei diesem wird gemäß vorliegender Erfindung vorzugsweise zunächst das gewünschte Makrodiol (oder auch mehrere verschiedene) in Lösung in einem geeigneten wasserfreiem organischen Lösungsmittel mit etwas mehr als der doppelten molaren Menge an Diisocyanat umgesetzt, um beidseitig Isocyanat-terminierte Präpolymere oder Zwischenprodukte mit der Kettenstruktur I-M-I zu erhalten, wonach die beiden, die Kettenverlängerer $C_1$ und $C_2$ einführenden Reaktanten nacheinander in molaren Mengen zugesetzt werden, deren Summe der molaren Menge des Makrodiols entspricht.

Aufgrund der geringeren Reaktivität der sterisch gehinderten Amine und der Instabilität der von ihnen gebildeten Harnstoffbindungen, vor allem in Lösung, wird die Kettenverlängerer-Gruppierung $C_1$ vorzugsweise als letzter Baustein in die erfindungsgemäßen TPUUs eingeführt. Somit wird das Isocyanat-terminierte Präpolymere mit der Kettenstruktur

I-M-I in bevorzugten Ausführungsformen zunächst mit dem $C_2$ enthaltenden Diol, Diamin oder Aminoalkohol umgesetzt, was beispielsweise bei Reaktion der in Bezug auf die Makrodiol-Einheiten M halben molaren Menge an Kettenverlängerer Isocyanat-terminierte Zwischenprodukte mit der Kettenstruktur I-M-I-$C_2$-I-M-I ergibt. Diese werden anschließend mit dem $C_1$ enthaltenden Kettenverlängerer-Reagens, d. h. dem sterisch gehinderte Aminogruppen enthaltenden Diamin oder Aminoalkohol, umgesetzt, was bei äquimolaren Mengen der beiden Kettenverlängerer ein TPUU-Polymer mit Blöcken einer Struktur der Formel (V) ergibt:

$$-[I-M-I-C_1-I-M-I-C_2]_n- \qquad (V)$$

d. h. TPUUs der Formel (I), worin a = b = c = 1 ist. Der Wert von n, d. h. die Anzahl an Blöcken, und damit die Kettenlänge und das Molekulargewicht der TPUUs hängen dabei neben der Reinheit der Monomere, wie zuvor erwähnt, vor allem von deren Stöchiometrie sowie von der Reaktionsführung und dabei vor allem von der Reihenfolge des Zusatzes der verschiedenen Monomer- oder Präpolymer-Komponenten ab. Vorzugsweise ist $n \geq 5$, noch bevorzugter $\geq 10$, noch bevorzugter $\geq 20$.

Die obige Reaktionsführung, beginnend mit der Herstellung beidseitig Isocyanat-terminierte Zwischenprodukte mit der Kettenstruktur I-M-I und anschließender aufeinanderfolgender Reaktion mit den beiden Kettenverlängerer-Komponenten ist zwar gemäß vorliegender Erfindung bevorzugt, aber Letztere ist nicht darauf eingeschränkt. Beispielsweise könnte auch ein Gemisch der beiden Kettenverlängerer-Komponenten mit einer der Summe der beiden molaren Mengen entsprechenden (bzw. diese geringfügig übersteigenden) Menge an Diisocyanat umgesetzt werden, um ein Präpolymer herzustellen, in dem nur Kettenverlängerer-Bausteine $C_1$ und $C_2$ über Diisocyanat-Bausteine I verbunden und alternierend angeordnet sind. Diese können dann mit Makrodiol und weiterem Diisocyanat zu den gewünschten TPUU-Ketten umgesetzt werden.

Allerdings würden in diesem Fall bei Verwendung von primären Aminen zur Einführung der $C_2$-Einheiten aufgrund der relativen Reaktionsträgheit der sterisch gehinderten Amine im Vergleich zu primären Aminen zunächst vor allem die Einheiten $C_2$ über Diisocyanate verknüpft werden, bevor die Mehrzahl der Einheiten $C_1$ in die Ketten eingebaut wird, so dass Letztere statistisch häufiger an den Enden der Präpolymerketten anzutreffen wären. Dies ist jedoch nicht zu bevorzugen, da diese Strategie zu Häufungen von sterisch gehinderten Harnstoffgruppierungen an manchen Stellen der endgültigen TPUU-Ketten, nämlich jeweils an den Enden der die Kettenverlängerer enthaltenden Hartsegmente, führt, was die Wirkung der vorliegenden Erfindung nicht verbessern würde und daher eher unwirtschaftlich wäre. Dazu kommt, dass es bei solchen Polymerketten, die darüber hinaus relativ niedrige Molekulargewichte, d. h. niedrige Werte für n, aufweisen, bei der späteren Wasserbehandlung inmitten der Polymerketten kaum zu einer Erhöhung der intermolekularen Bindungen aufgrund der Ausbildung von Wasserstoffbrücken durch neu entstandene, nicht sterisch gehinderte Harnstoffgruppierungen kommt. Relativ zu Alkoholen sind sterisch gehinderte Amine jedoch reaktiver. Werden daher Diole zur Einführung der $C_2$-Einheiten eingesetzt, sollten diese, gegebenenfalls bei erhöhten Temperaturen und/oder unter Verwendung von Katalysatoren, zuerst in Präpolymerketten eingebaut werden, bevor diese mit den sterisch gehinderten Aminen zu den endgültigen TPUU-Ketten umgesetzt werden, da es ansonsten zu unerwünschten Häufungen von sterisch gehinderten Harnstoffgruppierungen inmitten der Hartsegemente oder mitunter sogar zu einem unvollständigen Einbau der Einheiten $C_2$ kommen könnte.

Aus diesen Gründen ist gemäß vorliegender Erfindung die obige, mit der Herstellung beidseitig Isocyanat-terminierter Zwischenprodukte der Struktur I-M-I beginnende und mit getrennten Reaktionen mit den beiden jeweiligen Kettenverlängerer-Komponenten fortgesetzte Reaktionsführung zu bevorzugen. Weiters werden TPUUs bevorzugt, die der obigen Formel (IV) und insbesondere der Formel (V) entsprechen, da in diesen Fällen die Abstände zwischen den Kettenverlängerer-Einheiten $C_1$ und $C_2$ anhand der Länge des Makrodiols gut steuerbar sind.

Das zur Herstellung der TPUUs eingesetzte organische Lösungsmittel ist nur insofern eingeschränkt, als es wasserfrei, d. h. absolut, gegenüber der Polyadditionsreaktionen inert, d. h. aprotisch, und in der Lage sein muss, die Monomere und die daraus gebildeten Präpolymere sowie vorzugsweise auch die gewünschten Polymere zu lösen, um hohe Molekulargewichte der Letzteren zu ermöglichen, zu welchem Zweck es erforderlichenfalls auch erhitzt werden kann. Geeignete Beispiele umfassen aprotische polare Lösungsmittel wie Aceton, Acetonitril, Tetrahydrofuran (THF), Dimethylformamid (DMF), Dimethylsulfoxid (DMSO), N-Methylpyrrolidon (NMP) und Gemische davon. Auch die Temperaturen für sämtliche Teilreaktionen der Polyaddition sind nicht speziell eingeschränkt, hängen ebenfalls von der Reaktivität der Monomere ab und reichen von Raumtemperatur bis zur Rückflusstemperatur des jeweiligen Lösungsmittels. Bevorzugte Temperaturbereiche sind beispielsweise 30-120 °C, 40-80 °C oder 50-70 °C.

Aufgrund der Reaktivität der erfindungsgemäßen TPUUs mit Wasser ist während der gesamten Polyaddition Wasserfreiheit des Reaktionssystems zu gewährleisten, wozu neben der Verwendung von absoluten Lösungsmitteln beispielsweise auch die Anwendung der bekannten Schlenktechnik und/oder andere Vorkehrungen zum Ausschluss von Luftfeuchtigkeit zählen, mit denen der Durchschnittsfachmann wohlvertraut ist.

Die Reinigung der TPUUs kann auf bekannte, allgemein übliche Weise erfolgen, beispielsweise durch einfache Ausfällung aus der Reaktionslösung, mitunter gefolgt von einer Umfällung aus demselben oder einem anderen Lösungsmittel. Für Fachleute auf dem Gebiet der Polymerchemie zählen entsprechende Vorgangsweisen zum Standardrepertoire.

In einem zweiten Aspekt betrifft die Erfindung auch die Verwendung der thermoplastischen Poly(urethanharnstoff)-Polyaddukte als bei Kontakt mit Wasser selbstverstärkende Polymere in thermomechanischen oder lösungsbasierten Verarbeitungsverfahren, wobei sie vorzugsweise zu einem festen Erzeugnis verarbeitet werden, das während oder nach der Verarbeitung Wasser oder einem wässrigen Milieu ausgesetzt wird, um eine oder mehrere seiner thermomechanischen Eigenschaften zu verbessern. Beispiele für solche Verarbeitungsverfahren sind sämtliche eingangs genannten, darunter Folienziehen und andere thermische und lösungsbasierte Fertigungsverfahren.

Feste Erzeugnisse, die so aus den erfindungsgemäßen TPUUs erhalten wurden, insbesondere aus solchen mit unter physiologischen Bedingungen spaltbaren Estergruppierungen und/oder aus solchen, deren Reste I, M, $C_1$ und $C_2$ sowie etwaige Spaltprodukte davon physiologisch unbedenklich sind, sind vorzugsweise als Biomaterial in biomedizinischen Anwendungen einsetzbar, wo biologische Abbaubarkeit vor großem Vorteil ist. In besonders bevorzugten Ausführungsformen der Erfindung wird das thermoplastische Poly(urethan-harnstoff)-Polyaddukt daher zur Herstellung von temporären Körperimplantaten eingesetzt bzw. ist das daraus erhaltene feste Erzeugnis als temporäres Körperimplantat einsetzbar.

Die Verbesserung der thermomechanischen sowie anderer Eigenschaften der erfindungsgemäßen TPUUs kann dabei entweder vor oder nach dem Implantieren erfolgen. So kann etwa das TPUU-Material durch eines der hierin genannten Verfahren - oder auch durch ein anderes, bei dem die verbesserten Eigenschaften von Vorteil sind - zu einem festen Erzeugnis verarbeitet werden, das zur Verwendung als Körperimplantat bestimmt ist, und vor dem Implantieren für eine bestimmte Zeitspanne einer wässrigen Umgebung ausgesetzt werden, um "Rekombinationsreaktionen" analog zu obigem Schema D zu bewirken. Oder das feste Erzeugnis wird in dem bei der Verarbeitung erhaltenen Zustand in den Körper eines Patienten implantiert, wonach es durch die wässrige Umgebung unter physiologischen Bedingungen die genannten Reaktionen und die damit einhergehenden Eigenschaftsverbesserungen erfährt. In beiden Fällen erfährt es weiters während seines Verbleibs im Körper eine Spaltung der in den erfindungsgemäßen TPUUs enthaltenen Esterbindungen, wodurch die Polymerketten im Körper nach und nach, z. B. über einen Zeitraum von mehreren Monaten oder Jahren, zur Gänze abgebaut werden.

## KURZBESCHREIBUNG DER ZEICHNUNGEN

Die vorliegende Erfindung wird nachstehend anhand von illustrativen, nichteinschränkenden Ausführungsbeispielen und unter Bezugnahme auf die beiliegenden Zeichnungen näher beschrieben, die Folgendes zeigen:

Fig. 1 einen grafischen Vergleich der Zugdehnung von aus dem in Beispiel 1 hergestellten TPUU der vorliegenden Erfindung gezogenen Folien nach 24-stündiger Lagerung in trockenem Zustand und in Wasser;

Fig. 2 einen grafischen Vergleich der Zugfestigkeit von aus den in den Beispielen 1 bis 3 hergestellten TPUUs der vorliegenden Erfindung und aus bekannten TPUs gezogenen Folien nach 7-tägiger Lagerung in trockenem Zustand und in Wasser; und

die Fig. 3 bis 5 die Ergebnisse eines Experiments zur Untersuchung der Abbaubarkeit der erfindungsgemäßen TPUUs unter simulierten physiologischen Bedingungen.

## BEISPIELE

Als repräsentative Beispiele für besonders bevorzugte Ausführungsformen der vorliegenden Erfindung wurden mehrere TPUUs unter Anwendung der zuvor dargelegten bevorzugten Reaktionsführung hergestellt, d. h. durch aufeinanderfolgende Reaktion der einzelnen Komponenten unter anfänglicher Herstellung von beidseitig Isocyanatterminierten Präpolymeren bzw. Zwischenprodukten mit der Kettenstruktur I-M-I, die nacheinander mit den beiden, die Kettenverlängerer $C_2$ und danach $C_1$ einführenden Reaktanten umgesetzt wurden. Letztere wurden dabei in unterschiedlichen Molverhältnissen eingesetzt, die Summe der molaren Mengen entsprach aber jeweils der Molmenge an Makrodiol.

Auf diese Weise wurden mehrere bevorzugte erfindungsgemäße TPUUs der Formel (I) erhalten:

$$-[I\text{-}M\text{-}(I\text{-}C_1)_a\text{-}(I\text{-}M)_b\text{-}(I\text{-}C_2)_c]_n\text{-} \qquad (I)$$

worin b + 1 = a + c ist, genauer gesagt TPUUs der Formel (IV):

$$-[(I\text{-}M\text{-}I\text{-}C_1)_a\text{-}(I\text{-}M\text{-}I\text{-}C_2)_c]_n\text{-} \qquad (IV)$$

worin a und c jeweils aus 1 und 3 ausgewählt sind und worin n ≥ 10 oder n ≥ 20 ist.

**Beispiel 1**

Unter Anwendung der Standard-Schlenktechnik mit Argon als Inertgas wurde zunächst vorgetrocknetes Polytetrahydrofuran (pTHF) ($M_n$ ≈ 1 kDa, 6,059 g, 6,1 mmol, 1,00 Äqu., 19 ppm $H_2O$) als Makrodiol in einen Reaktionskolben eingewogen und eine weitere Stunde lang bei 60 °C im Hochvakuum getrocknet. Anschließend wurden 5 ml abs. Dimethylformamid (DMF) und danach Hexamethylendiisocyanat (HMDI) (2,111 g, 12,6 mmol, 2,07 Äqu.) in 5 ml abs. DMF zu dem trockenen, geschmolzenen pTHF zugesetzt. Nach der Zugabe von 2 Tropfen (ca. 0,04 ml) Zinn(II)-2-ethylhexanoat als Katalysator wurde das Reaktionsgemisch unter Argon-Schutzatmosphäre 3 h lang bei 60 °C magnetisch gerührt. Anschließend wurde Bis(hydroxyethyl)terephthalat (BHET) (0,770 g, 3,03 mmol, 0,5 Äqu.) als Diol zur Einführung von $C_2$ in Form einer Lösung in 5 ml abs. DMF zugesetzt. Nach weiteren 3 h Rühren bei 60 °C wurde das Reaktionsgemisch auf Raumtemperatur abgekühlt, wonach N,N'-Di-tert-butylethylendiamin (TBEDA) (0,522 g, 3,03 mmol, 0,5 Äqu.) als beidseitig sterisch gehindertes sekundäres Diamin zur Einführung von $C_1$ zusetzt wurde. Nach jeder Zugabe wurden die Transfer-Gefäße bzw. -Spritzen jeweils mit 5 ml abs. DMF gespült. Die Reaktionslösung wurde über Nacht weitergerührt. Zur Gewinnung und Reinigung des hergestellten TPUU wurde das Reaktionsgemisch mit DMF verdünnt und zum zehnfachen Volumen an Diethylether zugetropft und dabei als farbloser Niederschlag ausgefällt, der anschließend getrocknet wurde.

Durch diese Umsetzung der Reaktanten im Verhältnis $C_1 : C_2 : M : I = 1 : 1 : 2 : 4$ (bzw. 4,14) wurde ein besonders bevorzugtes erfindungsgemäßes TPUU der obigen Formel (IV) erhalten, worin a = b = c = 1 ist, d. h. ein TPUU der Formel (V):

$$-[I\text{-}M\text{-}I\text{-}C_1\text{-}I\text{-}M\text{-}I\text{-}C_2]_n- \qquad (V)$$

Der Wert für n wurde aus dem mittels Gelpermeationschromatographie (GPC) bestimmten gewichtsmittleren Molekulargewicht ($M_w$) und der Molmasse der Blöcke berechnet. Für $M_w$ des erhaltenen TPUU wurden rund 65,6 kDa ermittelt, und die Molmasse eines Blocks betrug rund 3,1 kDa, woraus ein Wert für n von rund 21 folgt.

Die genaue Struktur dieses in Beispiel 1 erhaltenen TPUU der Formel (V) ist umseitig dargestellt. Der durchschnittliche Wert für m der von PolyTHF mit einem zahlenmittleren Molekulargewicht $M_n$ von etwa 1 kDa stammenden Einheiten M beträgt darin rund 14. Unterhalb sind weiters die den Einheiten $C_1$, $C_2$, M und I entsprechenden Abschnitte angegeben.

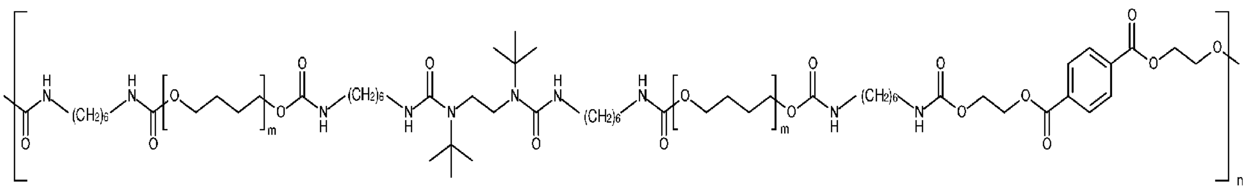

I M I $C_1$ I M I $C_2$

**Beispiel 2**

Zur Herstellung einer weiteren Ausführungsform der erfindungsgemäßen TPUUs wurde Beispiel 1 im Wesentlichen wiederholt, wobei jedoch das Molverhältnis zwischen den nacheinander in die Polymerketten eingebauten Kettenverlängerer-Einheiten $C_2$ und $C_1$ von 1:1 auf 3:1 geändert wurde. Das heißt, es wurden zunächst statt 0,5 Äquivalenten Bis(hydroxyethyl)terephthalat 0,75 Äquivalente und danach statt 0,5 Äquivalenten N,N'-Di-tert-butylethylendiamin nur 0,25 Äquivalente umgesetzt.

Durch die Umsetzung der vier Reaktanten im Verhältnis $C_1 : C_2 : M : I = 0{,}5 : 1{,}5 : 2 : 4$ (bzw. 4,14) wurde ein TPUU der Formel (IV) erhalten:

$$-[(I\text{-}M\text{-}I\text{-}C_1)_a\text{-}(I\text{-}M\text{-}I\text{-}C_2)_c]_n- \qquad (IV)$$

worin a = 1 ist und c = 3 ist. Die vier jeweils einen der beiden Kettenverlängerer enthaltenden Abschnitte sind dabei innerhalb eines Blocks statistisch verteilt.

Für das $M_w$ des so erhaltenen TPUU wurden mittels GPC rund 62,8 kDa ermittelt, und die Molmasse eines Blocks betrug rund 6,3 kDa, woraus ein Wert für n von rund 10 folgt.

**Beispiel 3**

Zur Herstellung einer weiteren bevorzugten Ausführungsform eines erfindungsgemäßen TPUU wurde Beispiel 2 im Wesentlichen wiederholt, wobei in diesem Fall das Molverhältnis zwischen $C_2$ und $C_1$ umgekehrt wurde. Das heißt, es wurden zunächst statt 0,5 Äquivalenten Bis(hydroxyethyl)terephthalat nur 0,25 Äquivalente und danach statt 0,5 Äquivalenten N,N'-Di-tert-butylethylendiamin 0,75 Äquivalente umgesetzt.

Durch die Umsetzung der vier Reaktanten im Verhältnis $C_1 : C_2$: M : I = 1,5 : 0,5 : 2 : 4 (bzw. 4,14) wurde ein TPUU der Formel (IV) erhalten:

$$-[(I\text{-}M\text{-}I\text{-}C_1)_a\text{-}(I\text{-}M\text{-}I\text{-}C_2)_c]_n\text{-} \qquad (IV)$$

worin a = 3 ist und c = 1 ist und die vier die Kettenverlängerer enthaltenden Abschnitte innerhalb eines Blocks wiederum statistisch verteilt sind.

Für das $M_w$ des so erhaltenen TPUU wurden mittels GPC 74,4 kDa ermittelt, und die Molmasse eines Blocks betrug rund 6,1 kDa, woraus ein Wert für n von rund 12 folgt.

Vergleichsbeispiel 1

Auf analoge Weise zu Beispiel 1 wurde ein Vergleichspolymer durch Umsetzung von pTHF, HMDI und BHET hergestellt, wobei jedoch kein sterisch gehindertes sekundäres Diamin zur Einführung von $C_1$ zusetzt, sondern eine zur Menge an pTHF äquimolare Menge an BHET eingesetzt wurde. Als Resultat beträgt das Verhältnis der Reste im Polyaddukt $C_2$: M: I = 1 : 1 : 2, das somit ein thermoplastisches Polyurethan (TPU; ohne Harnstoffgruppierungen) der nachstehenden Formel (VI) darstellt:

$$-[I\text{-}M\text{-}I\text{-}C_2]_n\text{-} \qquad (VI)$$

Für das $M_w$ des so erhaltenen TPU wurden mittels GPC 46 kDa ermittelt, und die Molmasse eines Blocks betrug rund 1,6 kDa, woraus ein Wert für n von rund 29 folgt.

Dieses TPU ist zwar aufgrund der spaltbaren Esterbindungen in $C_2$ unter physiologischen Bedingungen abbaubar, weist aber natürlich keine selbstverstärkenden Eigenschaften auf.

**Vergleichsbeispiel 2**

Zum Vergleich der obigen TPUUs mit einem im Handel erhältlichen TPU, einem thermoplastischen Polyurethan ohne Harnstoffgruppierungen, wurde Pellethane® 2363-80A von Lubrizol LifeSciences erstanden. Dabei handelt es sich um ein aus Methylendi(phenylisocyanat) (MDI), Polytetrahydrofuran (pTHF) und 1,4-Butandiol hergestelltes, nicht biologisch abbaubares TPU mit einem Molekulargewicht $M_n$ von etwa 37 kDa und einem $M_w$ von rund 63 kDa, das denselben Tests unterzogen wurde wie die TPUUs der erfindungsgemäßen Beispiele und jenes von Vergleichsbeispiel 1.

**Beispiel 4**

Herstellung und Testung von Folien

Die in den Beispielen 1 bis 3 hergestellten TPUUs der vorliegenden Erfindung sowie die TPUs der Vergleichsbeispiele 1 und 2 wurden in einer Konzentration von 10 Gew.-% in abs. DMF gelöst. Diese Lösungen wurden in Teflon-Formen mit den Abmessungen 60 x 40 x 2 mm gegossen, und das Lösungsmittel wurde bei Raumtemperatur abdampfen gelassen. Nach 24 h wurden die so erhaltenen Folien weitere 3 d lang im Exsikkator unter Vakuum getrocknet, wonach ihre Dicke mittels eines elektronischen Außenmessgeräts K110T von Kroeplin gemessen wurde und in allen Fällen rund 100 μm betrug. Je eine Hälfte der Folien wurde daraufhin in entionisiertes Wasser eingelegt und genau wie die jeweils andere Hälfte in trockenem Zustand insgesamt 7 d lang bei Raumtemperatur gelagert. Sämtliche nass gelagerten Proben wurden vor der Durchführung jeglicher Tests 24 h lang im Exsikkator bei 80 °C und 120 mbar getrocknet.

*Zugversuche*

Nach 24 h Nasslagerung und Trocknung wurden Abschnitte der beiden Folien aus dem in Beispiel 1 hergestellten TPUU als Zugversuchsproben vom Typ 5B ausgestanzt und einem Zugversuch gemäß ISO 527-1 unter Verwendung einer Zwick Z050-Zugprüfmaschine unterzogen, indem die in die Prüfmaschine eingespannten Proben mit einer Geschwindigkeit von 50 mm/min auseinandergezogen wurden, bis sie rissen. In Fig. 1 ist das Ergebnis dieses Versuchs

grafisch dargestellt. Ein Vergleich der tolerierten Maximal-Standardkraft der trockenen und der nassen Probe wird hierin als Maß für die Selbstverstärkung der erfindungsgemäßen TPUUs herangezogen. In Fig. 1 ist zu erkennen, dass die trocken gelagerte Folie bei einer Dehnung von rund 800 %, also beim etwa 9-Fachen ihrer Ursprungslänge, riss, während die 24 h lang in Wasser gelagerte Folie sogar einer über 1000%igen Dehnung, d. h. auf das 11-Fache ihrer Ausgangslänge, standhielt.

Nach 7 d wurden von jeder der trocken und nass gelagerten (und getrockneten) Folien der Beispiele 1 bis 3 und der Vergleichsbeispiele 1 und 2 jeweils drei Zugversuchsproben ausgestanzt und Zugversuchen wie oben beschrieben unterzogen. In Fig. 2 sind die dabei erhaltenen (und aus den Dreifachbestimmungen gemittelten) Ergebnisse grafisch dargestellt.

Hier ist zu erkennen, dass alle drei erfindungsgemäßen TPUUs durch die 7-tägige Lagerung in Wasser eine Verbesserung der Zugfestigkeit erfahren hatten. Diese war beim TPUU aus Beispiel 2, das die geringste Anzahl an labilen Harnstoffbindungen der Kettenverlängerer-Gruppierungen $C_1$ enthielt und folglich durch die Rekombinationsreaktionen bei Kontakt mit Wasser (analog zu Schema D) neue Polymere mit den größten Kettenlängen ergab, am stärksten ausgeprägt, während die TPUUs der Beispiele 1 und 3, die die doppelte bzw. die dreifache Menge an $C_1$-Einheiten enthielten, ein entsprechend weniger starkes Ausmaß an Verbesserung erfahren hatte. Nichtsdestotrotz war selbst bei dem TPUU aus Beispiel 3, das dreimal so viele labile Harnstoffbindungen wie jenes aus Beispiel 2 aufwies und daher die größte Anzahl an Rekombinationsreaktionen innerhalb der ursprünglichen Polymerkette erfahren haben musste, die Zugfestigkeit gegenüber dem entsprechenden trocken gelagerten Polymer um rund 50 % verbessert.

Im Gegensatz dazu wies das TPU aus Vergleichsbeispiel 1 ohne labile Harnstoffbindungen nach trockener und nasser Lagerung praktisch dieselbe Zugfestigkeit auf, während jene des TPUs von Vergleichsbeispiel 2, die im trockenen Zustand ein Vielfaches der übrigen getesteten Polymere betrug, durch die Lagerung in Wasser sogar um rund ein Drittel abgenommen hatte. Dies wurde von den Erfindern, ohne sich auf eine Theorie festlegen zu wollen, auf ein durch Quellung ausgelöstes Aufbrechen von Wasserstoffbrückenbindungen zwischen Urethangruppierungen benachbarter Polymerketten während der Lagerung in wässrigem Milieu zurückgeführt. Dadurch lag die so verschlechterte Zugfestigkeit des TPUs von Vergleichsbeispiel 2 im Bereich der durch die nasse Lagerung verbesserten Zugfestigkeit des erfindungsgemäßen TPUUs aus Beispiel 2.

*Schmelzpunktbestimmung*

Weiters wurden die Schmelzpunkte der in den Folien enthaltenen erfindungsgemäßen TPUUs mittels DSC bestimmt. Die gemessenen Werte sind in der nachstehenden Tabelle 1 angeführt.

Tabelle 1

| Beispiel | Verhältnis $C_1$:$C_2$ | Tmax-trocken (°C) | Tmax-nass (°C) | $\Delta$T(°C) |
|---|---|---|---|---|
| 1 | 1:1 | 80,41 | 85,81 | 5,40 |
| 2 | 1:3 | 87,44 | 92,26 | 4,82 |
| 3 | 3:1 | 75,38 | 84,41 | 9,03 |

Diese Werte zeigen, dass für die durch die Reaktion mit Wasser "rekombinierten" Polymere durchwegs eine Erhöhung der Schmelzpeak-Temperaturen festzustellen war, wobei das TPUU aus Beispiel 3 mit dem höchsten Anteil an labilen Harnstoffbindungen in $C_1$ zwar den niedrigsten Schmelzpunkt aufwies, dieser aber die stärkste Erhöhung durch die Rekombinationsreaktionen in Wasser erfahren hatte. Dementsprechend zeigte das TPUU aus Beispiel 2 mit den wenigsten $C_1$-Einheiten innerhalb der Polymerkette den höchsten Schmelzpunkt, der aufgrund der geringsten Anzahl an Rekombinationsreaktionen im Wasser auch die geringste Erhöhung erfuhr.

**Beispiel 5**

Abbaubarkeit

Zur Untersuchung, ob die neuen Polyaddukte der vorliegenden Erfindung unter physiologischen Bedingungen abbaubar sind, wurde aus dem in Beispiel 1 hergestellten TPUU mit einem Verhältnis der Kettenverlängerer-Einheiten $C_1$:$C_2$ = 1:1 auf analoge Weise wie in Beispiel 4 eine Folie hergestellt, allerdings in diesem Fall mit einer mittleren Dicke von 800 $\mu$m. Aus dieser Folie wurden 15 kreisförmige Scheiben mit einem Durchmesser von 5 mm ausgestanzt, deren Gewicht genau bestimmt wurde, das jeweils zwischen 15 und 20 mg betrug.

Anschließend wurde als Simulation physiologischer Bedingungen jeweils eine Scheibe in einem Reagenzglas

in 20 ml PBS (1X, pH 7,4) eingelegt, wonach die Reagenzgläser in einem Autoklaven auf 90 °C erhitzt wurden. Nach 7, 14, 25, 35 und 41 d wurden jeweils drei davon entnommen. Die darin enthaltenen Scheiben wurden je dreimal für 15 min in entionisiertes Wasser eingelegt, um die enthaltenen Salze zu entfernen. Anschließend wurden sowohl das Abtropfgewicht als auch - nach Trocknung bis zur Gewichtskonstanz (24 h bei 80 °C und 120 mbar) - das Trockengewicht bestimmt sowie eine Molekulargewichtsbestimmung mittels Gelpermeationschromatographie durchgeführt. Aus den so erhaltenen Werten wurden anhand der nachstehenden Gleichungen 1 bis 3 der Massenverlust, die Molekulargewichtsabnahme und die Quellung der einzelnen Proben berechnet.

Gleichung 1: $$m_{eros}(t) = \frac{m_t - m_0}{m_0} \cdot 100$$

Gleichung 2: $$\%\overline{M}_w(t) = \frac{\overline{M}_w(t)}{\overline{M}_w(0)} \cdot 100$$

Gleichung 3: $$s(t) = \frac{m_t^w - m_t}{m_t} \cdot 100$$

$m_{eros}(t)$ Massenverlust nach t Tagen Abbau
t Abbau-Dauer in d
$m_t$ Probengewicht nach t Tagen Abbau in mg
$m_0$ Probengewicht vor dem Abbau in mg
$\%\overline{M}_w(t)$ Molekulargewichtsabnahme nach t Tagen Abbau in %
$\overline{M}_w(t)$ Molekulargewicht nach t Tagen Abbau in kDa
$\overline{M}_w(0)$ Molekulargewicht vor dem Abbau in kDa
s(t) Quellung der Probe nach t Tagen Abbau in %
$m_t^w$ Abtropfgewicht der Probe nach t Tagen Abbau in mg

Die Ergebnisse dieser Berechnungen sind in den Fig. 3 bis 5 grafisch dargestellt.

Daraus lässt sich vor allem ablesen, dass bereits nach 7 d eine Molekulargewichtsabnahme von rund 3/4 des Ausgangs-Molekulargewichts - bei rund 10%igem Massenverlust und kaum erhöhter Quellbarkeit - erfolgt war, die bis zum Ende der Abbaustudie nach 41 d - genau wie der Massenverlust - allmählich auf rund 90 % angestiegen war. Aus einer Extrapolation der Graphen der Fig. 3 und 4 lässt sich schließen, dass der Abbau nach etwa eine weitere Woche bei 90 °C in PBS, d. h. nach insgesamt rund 7 Wochen, wohl vollständig gewesen wäre.

Ein Vergleich der Anteile der Kettenverlängerer-Einheiten $C_1$ und $C_2$ des in diesem Beispiel getesteten TPUU aus Beispiel 1 mit jenen der beiden Polyaddukte der Beispiele 2 und 3, in denen der Anteil an spaltbaren Estergruppierungen in $C_2$ doppelt bzw. halb so groß ist, lässt die Schlussfolgerung zu, dass das TPUU aus Beispiel 2 wesentlich rascher und jenes aus Beispiel 3 deutlich langsamer abgebaut worden wären. Anzunehmen ist weiters, dass es keinen allzu großen Unterschied machen sollte, ob diese spaltbaren Estergruppierungen in den Einheiten $C_2$ oder in den Isocyanat-Einheiten I, in den Makrodiol-Einheiten M oder innerhalb der von sterisch gehinderten Aminen abgeleiteten Einheiten $C_1$ enthalten sind. Entsprechende Untersuchungen sind Gegenstand der derzeitigen Forschungen der Erfinder.

Zusammengefasst zeigen die obigen Ausführungen, dass die TPUUs der vorliegenden Erfindung unter simulierten physiologischen Bedingungen vollständig abbaubar sind und die Geschwindigkeit des Abbaus anhand der Anteile an spaltbaren Estergruppierungen steuerbar ist.

Somit wurde hierin belegt, dass die neuen erfindungsgemäßen thermoplastischen Poly(urethan-harnstoff)-Polyaddukte mit sterisch gehinderten Harnstoffgruppen der Formel (I) in festem Zustand durch eine Wasserbehandlung zu neuen Polymeren umsetzbar sind, deren physikalische Eigenschaften gegenüber jenen der Ausgangspolymere in mehrerlei Hinsicht verbessert sind. Aufgrund dessen eigenen sich die TPUUs der Erfindung hervorragend zur Herstellung von festen Erzeugnissen für verschiedenste Anwendungen. Aufgrund ihrer physiologischen Abbaubarkeit sind sie dabei insbesondere auch zur Verwendung als temporäre Körperimplantate geeignet.

**Patentansprüche**

1. Thermoplastisches Poly(urethan-harnstoff)-Polyaddukt mit sterisch gehinderten Harnstoffgruppen der nachstehen-

den Formel (I):

$$-[I\text{-}M\text{-}(I\text{-}C_1)_a\text{-}(I\text{-}M)_b\text{-}(I\text{-}C_2)_c]_n\text{-} \qquad (I)$$

worin I, M, $C_1$ und $C_2$ jeweils für zweiwertige Reste stehen, die jeweils über eine Urethan- oder eine Harnstoffgruppierung miteinander verbunden sind, wovon
I jeweils unabhängig für einen zweiwertigen, von einem Diisocyanat abgeleiteten, gesättigten oder ungesättigten, aliphatischen, alicyclischen oder aromatischen Rest mit 1 bis 20 Kohlenstoffatomen steht;
M jeweils unabhängig für einen zweiwertigen, von einem Makrodiol abgeleiteten Rest eines aliphatischen Polyethers, Polyesters oder Polycarbonats mit einem zahlenmittleren Molekulargewicht $M_n \geq 500$ steht;
$C_1$ jeweils unabhängig für einen zweiwertigen, von einem Diamin oder Aminoalkohol mit jeweils zumindest einer sterisch gehinderten sekundären Aminogruppe durch Entfernung je eines N-gebundenen Wasserstoffatoms des Diamins oder eines N-gebundenen und des O-gebundenen Wasserstoffatoms des Aminoalkohols abgeleiteten, gesättigten oder ungesättigten, aliphatischen oder alicyclischen Rest mit 1 bis 30 Kohlenstoffatomen steht;
$C_2$ jeweils unabhängig für einen zweiwertigen, von einem von einem Diol, Diamin oder Aminoalkohol abgeleiteten, gesättigten oder ungesättigten, aliphatischen, alicyclischen oder aromatischen Rest mit 1 bis 20 Kohlenstoffatomen steht;
wobei in den Resten von I, $C_1$ und $C_2$ bei Vorliegen von mehr als vier Kohlenstoffatomen gegebenenfalls zumindest eines davon durch ein aus Sauerstoff und Stickstoff ausgewähltes Heteroatom ersetzt ist;
wobei zumindest einer der Reste I, M, $C_1$ und $C_2$ eine oder mehrere Estergruppierungen umfasst; und
a, b und c jeweils unabhängig für eine ganze Zahl von 0 bis 10 stehen und n eine Zahl $\geq 3$ ist, die für die Anzahl der Blöcke des Polyaddukts steht;
mit der Maßgabe, dass jeweils innerhalb desselben Blocks $a + c \geq 1$ ist und in allen Blöcken zusammen zumindest ein $a \geq 1$ ist und zumindest ein $c \geq 1$ ist.

**2.** Thermoplastisches Poly(urethan-harnstoff)-Polyaddukt nach Anspruch 1, **dadurch gekennzeichnet, dass**:

a und c jeweils unabhängig $\leq 5$ oder $\leq 3$ sind; und/oder
a und c jeweils unabhängig $\geq 1$ sind; und/oder
$b \geq 1$ ist; und/oder
$b = c$ oder $b = a$ oder $b + 1 = a + c$ ist; und/oder
$n \geq 5$ oder $n \geq 10$ oder $n \geq 50$ ist.

**3.** Thermoplastisches Poly(urethan-harnstoff)-Polyaddukt nach Anspruch 1 oder 2, **dadurch gekennzeichnet, dass** zumindest ein Teil der Estergruppierungen unter physiologischen Bedingungen spaltbar ist und die Reste I, M, $C_1$ und $C_2$ sowie etwaige Spaltprodukte davon biokompatibel und physiologisch unbedenklich sind.

**4.** Thermoplastisches Poly(urethan-harnstoff)-Polyaddukt nach einem der Ansprüche 1 bis 3, **dadurch gekennzeichnet, dass** die Reste I jeweils unabhängig von einem Diisocyanat aus der aus den folgenden bestehenden Gruppe abgeleitet sind: 1,6-Hexamethylendiisocyanat, 4,4'-Diisocyanatodicyclohexylmethan, Isophorondiisocyanat, 1,3-Bis(isocyanatomethyl)cyclohexan, Diphenylmethan-4,4'-diisocyanat, L-Lysinethylesterdiisocyanat.

**5.** Thermoplastisches Poly(urethan-harnstoff)-Polyaddukt nach einem der Ansprüche 1 bis 4, **dadurch gekennzeichnet, dass** die Reste M jeweils unabhängig von einem Polyether, Polyester oder Polycarbonat aus der aus den folgenden bestehenden Gruppe abgeleitet sind: Polytetrahydrofuran, Polyethylenglykol, Polypropylenglykol, Polycaprolacton, Polylactid, Polyglykolid, Poly(lactid-co-glykolid), Polyhexamethylencarbonat.

**6.** Thermoplastisches Poly(urethan-harnstoff)-Polyaddukt nach einem der Ansprüche 1 bis 5, **dadurch gekennzeichnet, dass** die Reste $C_1$ jeweils unabhängig von einem Diamin abgeleitet und aus Resten der nachstehenden Formel (II) ausgewählt sind:

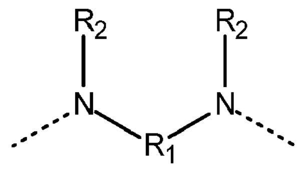

(II)

worin die gestrichelten Linien jeweils die Bindung an die Carbonylgruppe einer die Reste I, M, $C_1$ und $C_2$ verbindenden Urethan- oder Harnstoffgruppierung anzeigen;

$R_1$ aus zweiwertigen, gesättigten oder ungesättigten, aliphatischen oder alicyclischen Resten mit 1 bis 20 Kohlenstoffatomen ausgewählt ist; und
die $R_2$ jeweils unabhängig aus Wasserstoff sowie einwertigen, sperrigen, gesättigten oder ungesättigten, aliphatischen oder alicyclischen Resten mit 1 bis 10 Kohlenstoffatomen ausgewählt sind, mit der Maßgabe, dass nicht beide $R_2$ gleichzeitig Wasserstoff sind.

7. Thermoplastisches Poly(urethan-harnstoff)-Polyaddukt nach Anspruch 6, **dadurch gekennzeichnet, dass** $R_1$ aus $C_1$-$C_{10}$-Alkylen- und $C_4$-$C_{10}$-Cycloalkylenresten ausgewählt ist; und/oder
die $R_2$ jeweils unabhängig aus 1,1-Dimethyl-substituierten, gesättigten oder ungesättigten $C_1$-$C_6$-Alkylresten und 1-Methyl-substituierten $C_3$-$C_6$-Cycloalkylresten ausgewählt sind.

8. Thermoplastisches Poly(urethan-harnstoff)-Polyaddukt nach Anspruch 7, **dadurch gekennzeichnet, dass** $R_1$ aus $C_2$-$C_6$-Alkylen- und $C_5$-$C_6$-Cycloalkylenresten ausgewählt ist; und/oder
die $R_2$ jeweils unabhängig aus Isopropyl, tert-Butyl, 1,1-Dimethylpropyl und 1-Methylcyclohexyl ausgewählt sind.

9. Thermoplastisches Poly(urethan-harnstoff)-Polyaddukt nach einem der Ansprüche 1 bis 8, **dadurch gekennzeichnet, dass** zumindest einer der Reste $C_2$ eine oder mehrere Estergruppierungen umfasst.

10. Thermoplastisches Poly(urethan-harnstoff)-Polyaddukt nach Anspruch 9, **dadurch gekennzeichnet, dass** die Reste $C_2$ jeweils unabhängig von einem Diol aus der aus den folgenden bestehenden Gruppe abgeleitet sind: Bis(hydroxyethyl)terephthalat, Bis(hydroxypropyl)carbonat, 2-Hydroxyethyllactat und 2-Hydroxyethylglykolat.

11. Thermoplastisches Poly(urethan-harnstoff)-Polyaddukt nach einem der Ansprüche 1 bis 10, **dadurch gekennzeichnet, dass** b + 1 = a + c ist und das Polyaddukt der nachstehenden Formel (IV) entspricht:

$$-[(I\text{-}M\text{-}I\text{-}C_1)_a\text{-}(I\text{-}M\text{-}I\text{-}C_2)_c]_n- \qquad (IV)$$

worin
a und c jeweils unabhängig aus 1 bis 3 ausgewählt sind oder
a und c jeweils 1 sind; und
$n \geq 5$ oder $n \geq 10$ oder $n \geq 20$ ist.

12. Verwendung von thermoplastischen Poly(urethan-harnstoff)-Polyaddukten nach einem der Ansprüche 1 bis 11 als bei Kontakt mit Wasser selbstverstärkende Polymere in thermomechanischen oder lösungsbasierten Verarbeitungsverfahren.

13. Verwendung nach Anspruch 12, **dadurch gekennzeichnet, dass** das thermoplastische Poly(urethan-harnstoff)-Polyaddukt zu einem festen Erzeugnis verarbeitet wird, das während oder nach der Verarbeitung Wasser oder einem wässrigen Milieu ausgesetzt wird, um eine oder mehrere seiner thermomechanischen Eigenschaften zu verbessern.

14. Verwendung nach Anspruch 12 oder 13, **dadurch gekennzeichnet, dass**
die Reste I, M, $C_1$ und $C_2$ des thermoplastischen Poly(urethan-harnstoff)-Polyaddukts sowie etwaige Spaltprodukte davon biokompatibel und physiologisch unbedenklich sind; und
das thermoplastische Poly(urethan-harnstoff)-Polyaddukt bzw. das daraus erhaltene feste Erzeugnis als Biomaterial in biomedizinischen Anwendungen einsetzbar ist.

**15.** Verwendung nach Anspruch 14, **dadurch gekennzeichnet, dass**
zumindest ein Teil der Estergruppierungen des thermoplastischen Poly(urethan-harnstoff)-Polyaddukts unter physiologischen Bedingungen spaltbar ist; und
das thermoplastische Poly(urethan-harnstoff)-Polyaddukt zur Herstellung von temporären Körperimplantaten eingesetzt wird bzw. das daraus erhaltene feste Erzeugnis als temporäres Körperimplantat einsetzbar ist.

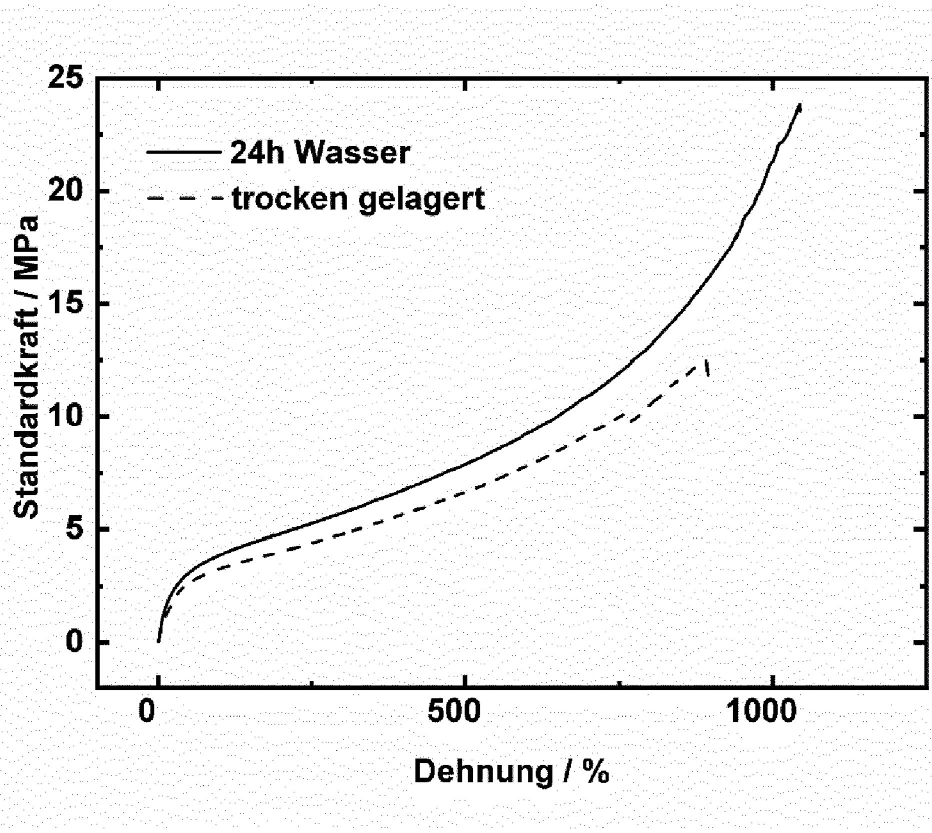
24h Wasser
trocken gelagert
Standardkraft / MPa
Dehnung / %
25
20
15
10
5
0
0
500
1000

Figur 1

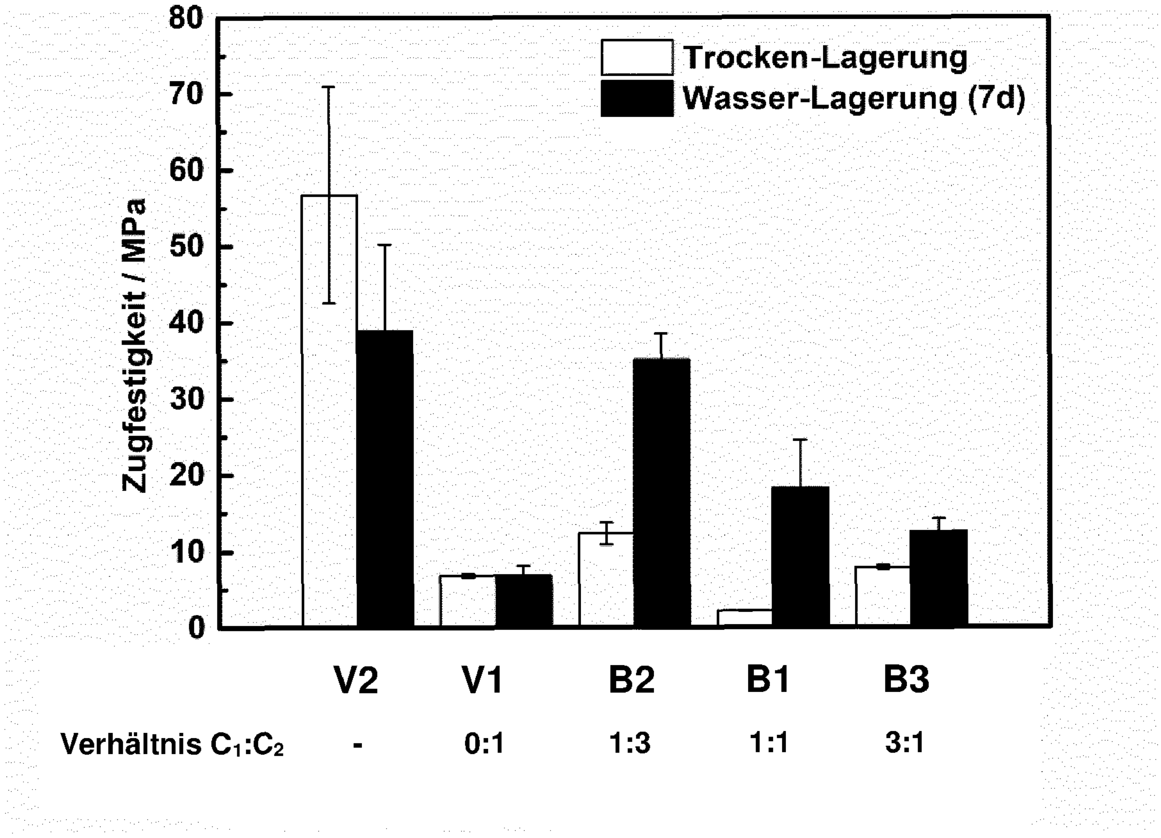

80
70
60
50
40
30
20
10
0
Zugfestigkeit / MPa
Trocken-Lagerung
Wasser-Lagerung (7d)
V2
V1
B2
B1
B3
Verhältnis C1:C2
-
0:1
1:3
1:1
3:1


Figur 2

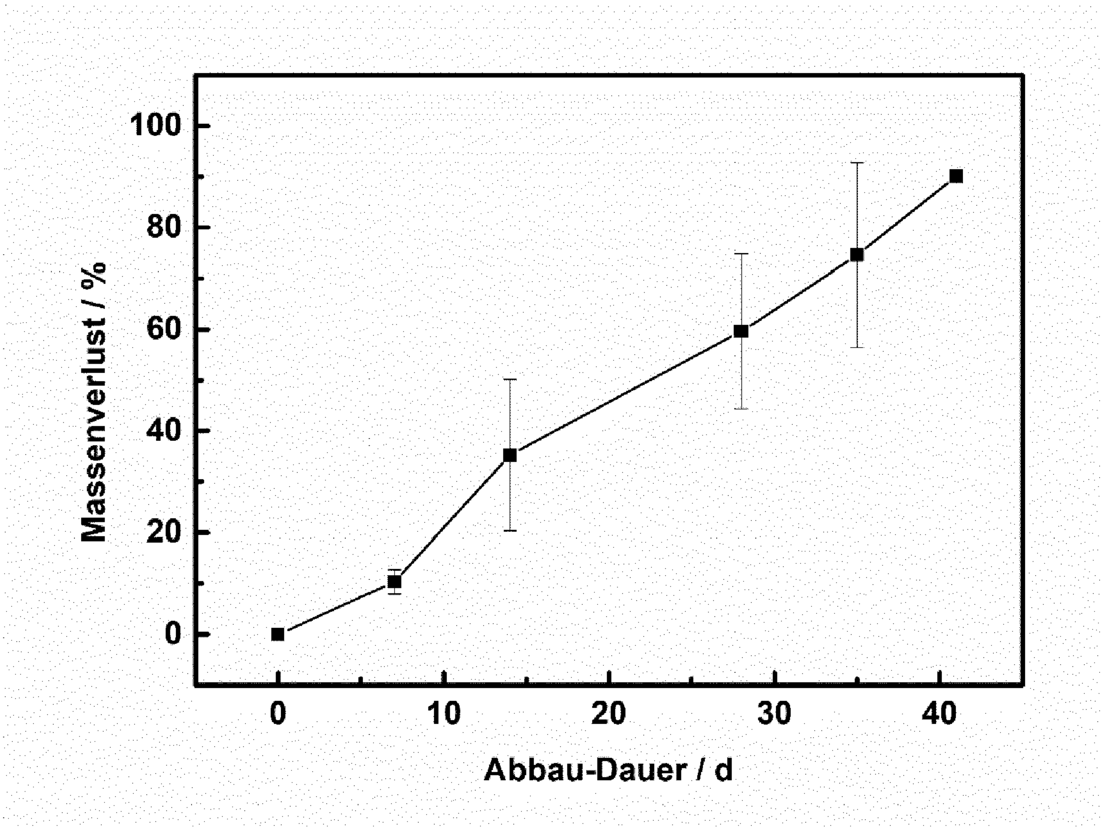


Figur 3

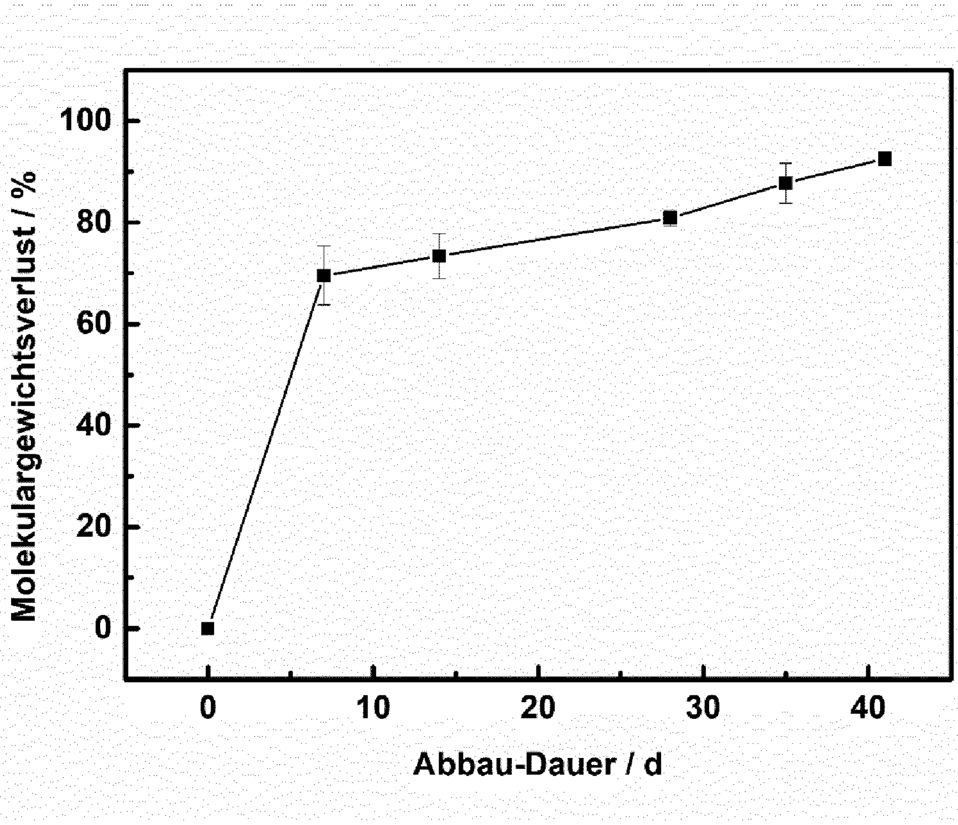
Molekulargewichtsverlust / %
100
80
60
40
20
0
0
10
20
30
40
Abbau-Dauer / d

Figur 4

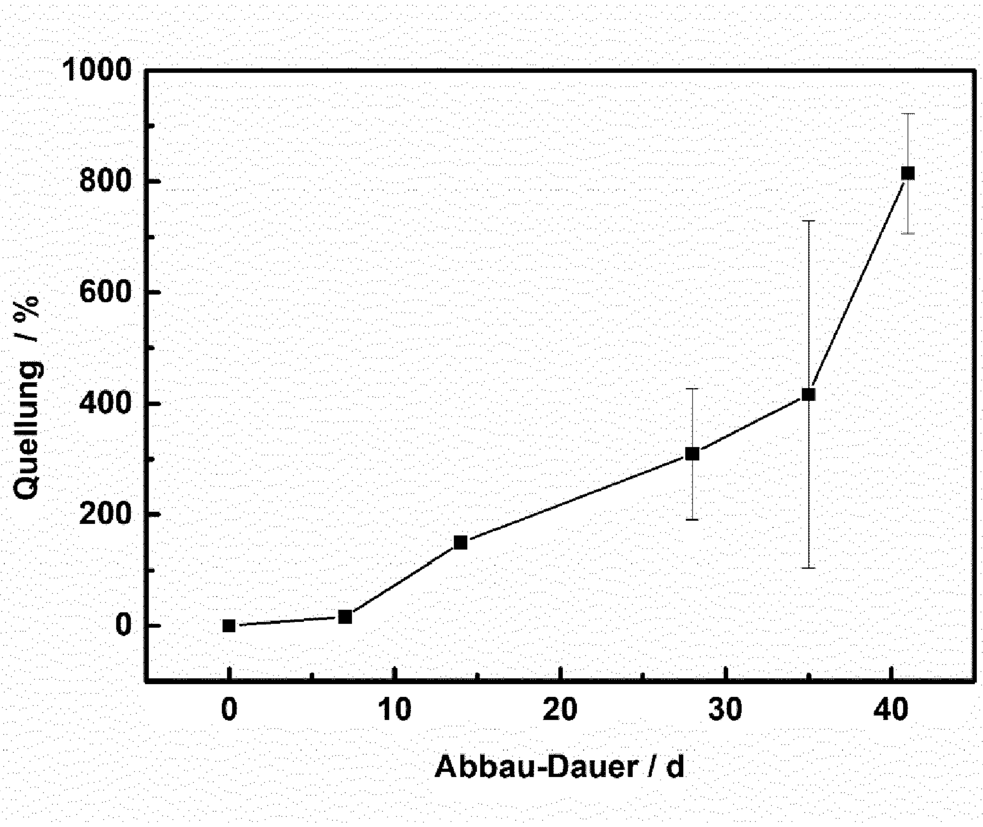


Figur 5

Europäisches Patentamt
European Patent Office
Office européen des brevets

# EUROPÄISCHER RECHERCHENBERICHT

Nummer der Anmeldung
EP 20 18 3957

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | EP 0 452 775 A2 (BAYER AG [DE])<br>23. Oktober 1991 (1991-10-23)<br>* Ansprüche 1-7; Beispiele 1-5 *<br>----- | 1-8,<br>12-14 | INV.<br>C08G18/73<br>C08G18/48<br>C08G18/10<br>C08G18/32<br>C09D175/02<br>C09D175/08<br>C08G18/22 |
| Y | WO 2005/089778 A1 (COMMW SCIENT IND RES ORG [AU]; ADHIKARI RAJU [AU] ET AL.)<br>29. September 2005 (2005-09-29)<br>* Seite 5, Zeile 29 - Seite 6, Zeile 1; Ansprüche 1-17 *<br>----- | 1-15 | |
| Y,D | HANZE YING ET AL: "Hydrolyzable Polyureas Bearing Hindered Urea Bonds",<br>JOURNAL OF THE AMERICAN CHEMICAL SOCIETY,<br>Bd. 136, Nr. 49,<br>26. November 2014 (2014-11-26), Seiten 16974-16977, XP055421622,<br>US<br>ISSN: 0002-7863, DOI: 10.1021/ja5093437<br>* scheme 1;<br>Seite 16974; Abbildungen 1-3 *<br>----- | 1-15 | |

RECHERCHIERTE SACHGEBIETE (IPC)
C08G
C09D

Der vorliegende Recherchenbericht wurde für alle Patentansprüche erstellt

1

| Recherchenort | Abschlußdatum der Recherche | Prüfer |
|---|---|---|
| München | 19. November 2020 | Scheuer, Sylvie |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 20 18 3957

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-11-2020

| Im Recherchenbericht angeführtes Patentdokument | Datum der Veröffentlichung | Mitglied(er) der Patentfamilie | Datum der Veröffentlichung |
|---|---|---|---|
| EP 0452775 A2 | 23-10-1991 | AT 129260 T | 15-11-1995 |
| | | CA 2040910 A1 | 21-10-1991 |
| | | DE 4012629 A1 | 24-10-1991 |
| | | EP 0452775 A2 | 23-10-1991 |
| | | JP H06145289 A | 24-05-1994 |
| WO 2005089778 A1 | 29-09-2005 | CN 1950098 A | 18-04-2007 |
| | | EP 1729783 A1 | 13-12-2006 |
| | | JP 5496457 B2 | 21-05-2014 |
| | | JP 2007530101 A | 01-11-2007 |
| | | MY 146259 A | 31-07-2012 |
| | | TW 200604249 A | 01-02-2006 |
| | | US 2006051394 A1 | 09-03-2006 |
| | | US 2015246994 A1 | 03-09-2015 |
| | | WO 2005089778 A1 | 29-09-2005 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 452775 A2
- WO 2014144539 A2
- WO 2016069582 A1
- WO 2016126103 A1
- WO 2016126756 A1
- WO 2017155958 A1


### In der Beschreibung aufgeführte Nicht-Patentliteratur


- **STOWELL, J. C. ; PADEGIMAS, S. J.** Urea dissociation. Measure of steric hindrance in secondary amines. *J. Org. Chem.,* 1974, vol. 39 (16), 2448-2449
- **HUTCHBY, M. ; HOULDEN, C. E. ; FORD, J. G. ; TYLER, S. N. G. ; GAGNE, M. R. ; LLOYD-JONES, G. C. ; BOOKER-MILBURN, K.I.** Hindered ureas as masked isocyanates: facile carbamoylation of nucleophiles under neutral conditions. *Angew. Chem. Int. Ed.,* 2009, vol. 48 (46), 8721-8724
- **YING, H. ; ZHANG, Y. ; CHENG, J.** Dynamic urea bond for the design of reversible and self-healing polymers. *Nat. Commun.,* 2014, vol. 5, 3218
- **ZHANG, Y. ; YING, H. ; HART, K. R. ; WU, Y. ; HSU, A. J. ; COPPOLA, A. M. ; KIM, T. A. ; YANG, K. ; SOTTOS, N. R. ; WHITE, S. R.** Malleable and recyclable poly-(urea-urethane) thermosets bearing hindered urea bonds. *Adv. Mater.,* 2016, vol. 28 (35), 7646-7651
- **ZHANG, L. ; ROWAN, S. J.** Effect of sterics and degree of cross-linking on the mechanical properties of dynamic poly(alkylurea-urethane) networks. *Macromolecules,* 2017, vol. 50 (13), 5051-5060
- **BRUCE, A. C. ; LEWIS, C. L.** Influence of glass transition temperature on mechanical and self-healing behavior of polymers bearing hindered urea bonds. *SPE ANTEC,* 2017
- **FANG, Z. ; ZHENG, N. ; ZHAO, Q. ; XIE, T.** Healable, reconfigurable, reprocessable thermoset shape memory polymer with highly tunable topological rearrangement kinetics. *ACS Appl. Mater. Interfaces,* 2017, vol. 9 (27), 22077-22082
- **WANG, Y. ; PAN, Y. ; ZHENG, Z. ; DING, X.** Reconfigurable and reprocessable thermoset shape memory polymer with synergetic triple dynamic covalent bonds. *Macromol. Rapid Commun.,* 2018, vol. 39 (10), 1800128
- **YING, H. ; CHENG, J.** Hydrolyzable polyureas bearing hindered urea bonds. *J. Am. Chem. Soc.,* 2014, vol. 136 (49), 16974-16977
- **YING, H. ; YEN, J. ; WANG, R. ; LAI, Y. ; HSU, J.-L.-A. ; HU, Y. ; CHENG, J.** Degradable and biocompatible hydrogels bearing a hindered urea bond. *Biomater. Sci.,* 2017, vol. 5 (12), 2398-2402
- **CAI, K. ; YING, H. ; CHENG, J.** Dynamic Ureas with fast and pHindependent hydrolytic kinetics. *Chem. Eur. J.,* 2018, vol. 24 (29), 7345-7348
- **CHEN, M. ; FENG, X. ; XU, W. ; WANG, Y. ; YANG, Y. ; JIANG, Z. ; DING, J.** PEGylated polyurea bearing hindered urea bond for drug delivery. *Molecules,* 2019, vol. 24 (8), 1538